# EUROPEAN PATENT APPLICATION

(11) **EP 3 399 053 A1**
(43) Date of publication of application: **07.11.2018**
(21) Application number: 18178441.4
(22) Date of filing: 13.03.2014
(51) Int. Cl.: C12Q 1/6806, G01N 33/53, C12Q 1/6841

(54) **CELL PREPARATIONS AND CELL SUPPORTS AND THEIR USE IN THERANOSIS**

(30) Priority: 14.03.2013 US 201361786062 P
(62) Divisional of application: 14717930.3
(71) Applicant: Abbott Molecular Inc., Des Plaines, IL 60018 (US)
(72) Inventor: SOKOLOVA, Irina, Villa Park, IL 60181 (US); SCHULZ, John, Arlington Heights, IL 60004 (US); KORNFELD, Olga, Glenview, IL 60025 (US); PANELLA, Jeffrey, Oaklawn, IL 60453 (US); SONG, Minghao, Lisle, IL 60532 (US); SITAILO, Svetlana, Brookfield, IL 60513 (US); POLICHT, Frank, Niles, IL 60714 (US); BROWNE, Gerry, Naperville, IL 60540 (US); PESTOVA, Ekaterina, Glenview, IL 60026 (US)
(74) Representative: Modiano, Micaela Nadia

(57) **Abstract**

A method of preparing cells from a patient specimen comprising a tissue or a clump of cells; a method of *in situ* hybridization (ISH) or immunohistochemistry (IHC); a method of theranosing a patient's response to an active agent that targets the tyrosine kinase pathway; an improvement of a method of theranosis comprising the method of ISH or IHC; a cell support on the surface of which are cells, which have been contacted with detectably labeled probes with the method of ISH or IHC; and a kit comprising (a) a cell support and/or at least one probe for ISH or IHC, and (b) instructions for carrying out the method of ISH or IHC on a cell support.

## Description

### CROSS-REFERENCE TO RELATED APPLICATIONS

This application claims priority to U.S. provisional patent application no. 61/786,062, which was filed on March 14, 2013, and which is hereby incorporated by reference in its entirety.

### TECHNICAL FIELD

The present disclosure relates to a method of preparing cells obtained from a tissue or clump of cells, cell supports comprising such cells, *in situ* hybridization (ISH), immunohistochemistry (IHC), the detection of chromosomal abnormalities, theranosis, and kits.

### BACKGROUND

Selection of targeted therapy for treating cancer, such as lung cancer, requires testing of a biopsy specimen with relevant cancer biomarkers in a short period of time. It is not unusual for there to be multiple, such as three or more, relevant biomarkers. A fine needle aspiration (FNA) or a core needle biopsy is oftentimes the only specimen obtained from a patient due to advanced stage of disease and accompanying sickness. Since the specimen contains only a small amount of cells, tissue, or biological fluid, such as bronchial aspiration in the case of lung cancer, it becomes critical to maximize use of the specimen.

Typically, an FNA or a core needle biopsy is processed in a manner similar to that of an excisional biopsy or a resection biopsy. The specimen is placed in formaldehyde for fixation for 24-48 hours. Subsequently, the fixed specimen is embedded in paraffin. Embedding a fixed specimen in paraffin can take an additional 24 hours. After the specimen has been fixed and embedded, the specimen is cut into sections of about 4-6 microns in thickness. The sections are then placed on slides for drying. Drying the sections can take up to another 24 hours. The slides, on the surfaces of which are the dried sections, need to be baked prior to fluorescence *in situ* hybridization (FISH). Baking the slides can take from about 2 hours to about 18 hours. Thus, preparing a specimen for FISH takes about five days. When a patient is in an advanced stage of disease or afflicted with an aggressive form of cancer, for example, time becomes a critical factor in patient outcome and survival.

Li et al. (Laboratory Investigation 86: 619-627 (2006)) attempted to improve cancer diagnosis by developing specific genomic probes for genes relevant to primary lung cancer and designing a multiple FISH array involving the arrangement of four different compositions of probes on a coverslip. Each composition contained a gene-specific probe and a centromeric probe for each of two chromosomes for a total of four probes, each of which was labeled with a different color. The coverslip was inverted over cells suspended in hybridization buffer on a slide. The method of Li et al. is disadvantageous in that it entails the generation of gene-specific genomic probes. In addition, the inversion of a zoned cover slip over a single suspension of cells can result in one or more zones of the cover slip being inverted over an area of the slide containing no cells or an insufficient number of cells for analysis. Furthermore, Li et al. only discloses the use of the method with bronchial washings and does not teach how to prepare other types of specimens for use with the method. Indeed, Li et al. indicates that the method is most likely to succeed in clinical specimens like bronchial washes and may not be suitable for small biopsy specimens or fixed tissues, where there is a risk of false negatives in one of the quadrants.

Abramovich et al. (RU2410663; Russian App. No. RU20090134364, published September 14, 2009) appears to disclose application of a cell suspension (5 µL) prepared by a standard method to holes formed by hermetic attachment of a perforated parafilm strip to the surface of a microscope slide. The parafilm strip is 26 x 56 mm² and contains eight holes, which are 9 mm in diameter, 4 mm apart, and 2 mm from the edge of the slide. The result is described as multiple, isolated zones for simultaneous FISH.

It is highly desirable to begin FISH within a matter of hours of specimen collection and to be able to analyze two or more, such as multiple, biomarkers simultaneously. Accordingly, it is an object of the present disclosure to provide a method of preparing cells for *in situ* hybridization (ISH), such as fluorescent ISH (i.e., FISH), in particular multiplexed FISH, and immunohistochemistry (IHC) within about one and a half hours of specimen collection, thereby enabling ISH and IHC results to be obtained in less than about 14 hours of specimen collection. This and other objects and advantages, as well as inventive features, will become apparent from the detailed description provided herein.

### SUMMARY

A method of preparing cells from a patient specimen comprising a tissue or a clump of cells is provided. The method comprises:
(i) soaking the patient specimen in a pre-warmed, balanced salt solution comprising a reducing agent and a chelating agent;
(ii) transferring the patient specimen to a fresh, balanced salt solution comprising a reducing agent, a source of calcium ions, and a polar aprotic solvent and mixing;
(iii) removing the patient specimen from the fresh, balanced salt solution and either (a) centrifuging the solution to pellet disaggregated cells or (b) straining the solution to collect disaggregated cells; and
(iv) after (iii)(a), discarding the supernatant, re-suspending the pelleted cells in residual supernatant, and placing the re-suspended cells in a fixative or, after (iii)(b), placing the strained cells in a fixative. The patient specimen can be (a) a fresh specimen, (b) a thawed, freshly frozen specimen, or (c) a partially fixed specimen. The method can further comprise rinsing the patient specimen in a balanced salt solution prior to (i).

An embodiment of the method comprises rinsing the patient specimen in Hank's buffer at room temperature, soaking the patient specimen in pre-warmed Hank's buffer containing 20 mM DTT (dithiothreitol) and 5 mM EDTA (ethylenediaminetetraacetic acid) at 37 °C for around five minutes, transferring the rinsed and soaked patient specimen to fresh Hank's buffer containing 20 mM DTT, 5 mM CaCl₂, and 10% DMSO (dimethylsulfoxide), mixing for about 5 seconds to about 10 seconds, removing the patient specimen from the fresh Hank's buffer and either (a) centrifuging the solution at about 1000 x g for about 10 minutes to pellet disaggregated cells or (b) straining the solution to collect disaggregated cells, and either, after centrifuging, discarding the supernatant, re-suspending the pelleted cells in residual supernatant, and placing the re-suspended cells in a solution or, after straining, placing the strained cells in a solution, wherein the solution comprises from about 30 wt.% to about 60 wt.% methanol, from about 40 wt.% to about 70 wt.% water, a buffer, and a preservative and wherein the solution is kept at room temperature overnight.

The method can further comprise (v) applying the cells to a cell support. If the cell support is a cytology slide, the method can further comprise (vi) soaking the slide in a water-alcohol solution, (vii) soaking the slide in a fixative, and (viii) drying the slide. The water-alcohol solution can be 95% ethanol, and the slide can be soaked in the ethanol for about 15 minutes at room temperature. The fixative can be Carnoy's solution, and the slide can be soaked in the Carnoy's solution for about 30 minutes at room temperature. The slide can be dried at room temperature.

A method of performing *in situ* hybridization (ISH) or immunohistochemistry (IHC) is also provided. In one embodiment, the method comprises contacting cells, which have been prepared in accordance with the above-described method, with a detectably labeled probe under hybridizing conditions and visualizing the detectable label in less than about 14 hours of removal of the specimen from the patient. In another embodiment, the method comprises contacting cells, which have been disaggregated from a specimen selected from the group consisting of a fine needle aspiration, a core needle biopsy, an excisional biopsy, and a resection, with a detectably labeled probe under hybridizing conditions and visualizing the detectable label in less than about 14 hours of removal of the specimen from the patient. The detectably labeled probe can be a fluorescently labeled nucleic acid probe, such as a probe for fluorescent ISH (FISH), or a fluorescently labeled antibody probe, such as a probe for immunohistochemistry (IHC). Preferably, the cells have been applied to a cell support. In a preferred embodiment, the cell support comprises at least two discrete areas and the cells have been applied to the discrete areas, in which case each area is contacted with at least one detectably labeled probe and, if an area is contacted with two or more detectably labeled probes, the detectably labeled probes can be distinguished. In an embodiment, each discrete area is separately contacted with a mixture of detectably labeled probes, wherein the mixture comprises:
--a two-color break-apart probe for ALK and a two-color break-apart probe for ROSI,
--a two-color break-apart probe for ALK, a two-color break-apart probe for ROS1, and a probe for c-MET,
--a two-color break-apart probe for ALK, a two-color break-apart probe for ROS1, and a two-color break-apart probe for RET,
--a two-color break-apart probe for RET and a two-color break-apart probe for NTRK1,
--a two-color break-apart probe for RET and a probe for KIF5B,
--a probe for MET, a probe for EGFR, and a CEP for chromosome 7,
--a probe for AURKA, a locus-specific probe for 9p21, a CEP for chromosome 3, a CEP for chromosome 6, and a CEP for chromosome 7,
--a probe for MYC, a probe for EGFR, a probe for DCC, a locus-specific probe for 13q14, and a CEP for chromosome 18,
--a probe for MYC, a probe for TERC, and a CEP for chromosome 7,
--a probe for MYC, a probe for HER2, a probe for ZNF217, and a locus-specific probe for 9p21,
--a probe for MCL1, a probe for FGFR2, a probe for MET, a probe for EGFR, and a CEP for chromosome 7,
--a probe for FGFR2, a probe for PIK3CA, a CEP for chromosome 3, and a CEP for chromosome 10,
--a probe for PTEN, a probe for ERG, and a CEP for chromosome 10,
--a probe for FGFR1, a probe for FGFR2, a two-color break-away probe for FGFR2, a CEP for chromosome 8, and a CEP for chromosome 10, or
--a probe for MET, a probe for PIK3CA, a probe for EGFR, a CEP for chromosome 7, and a CEP for chromosome 3.

Further provided is a method of theranosing a patient's response to an active agent that targets the tyrosine kinase pathway. The method comprises performing ISH or IHC according to an above-described method of using a cell support comprising at least two discrete areas, to which cells have been applied and which are separately contacted with at least one detectably labeled probe. If an area is contacted with two or more detectably labeled probes, the detectably labeled probes can be distinguished. The active agent that targets the tyrosine kinase pathway can be crizotinib, volitinib, gefitinib, erlotinib, sorafenib, sunitinib, dasatinib, trastuzumab, or pertuzumab.

Still further provided is an improvement of a method of theranosis. The improvement comprises the use of an above-described method of performing ISH or IHC, such as on a cell support comprising at least two discrete areas to which cells have been applied and which are contacted with at least one detectably labeled probe and, if an area is contacted with two or more detectably labeled probes, the detectably labeled probes can be distinguished.

Even still further provided is a cell support on the surface of which are cells, which have been contacted with detectably labeled probes, such as those described above, in accordance with an above-described method of performing ISH or IHC. The cell support comprises at least two discrete areas, in which case each area is contacted with at least one detectably labeled probe and, if an area is contacted with two or more detectably labeled probes, the detectably labeled probes can be distinguished. Each discrete area can be separately contacted with a mixture of detectably labeled probes, such as an above-described mixture.

Thus, also provided is a kit. The kit comprises (a) a cell support and/or at least one probe for ISH or IHC, and (b) instructions for carrying out an above-described method of performing ISH or IHC, such as on a cell support comprising at least two discrete areas to which cells have been applied and which are contacted with at least one detectably labeled probe and, if an area is contacted with two or more detectably labeled probes, the detectably labeled probes can be distinguished. Additionally or alternatively, the instructions can include instructions for carrying out an above-described method of theranosis.

### DETAILED DESCRIPTION

The present disclosure provides methods of preparing cells obtained from a tissue sample, methods of fluorescence *in situ* hybridization (FISH), in particular multiplexed FISH, immunohistochemistry (IHC), methods of theranosis, a cell support, and a kit. The methods of preparing cells enables nucleic acid hybridization (e.g., FISH, such as multiplexed FISH) to be performed as quickly as possible after a tissue specimen is obtained from a patient by fine needle aspiration (FNA), core needle biopsy, excisional biopsy, resection, and the like, such as in less than about 14 hours, e.g., about 13.5 hours. The tissue specimen may be (a) fresh, (b) thawed, freshly frozen, or (c) partially fixed. By avoiding the use of fixed and embedded specimens, hybridization results can be more clearly viewed and truncation artifacts associated with sectioning of the embedded specimens can be avoided. The method also enables a reduction, even a substantial reduction, in the volume of reagents, including probes that need to be used for ISH, such as FISH, as compared to ISH, such as FISH, on fixed and embedded specimens. Furthermore, the method enables ISH, such as FISH, to be performed in relatively small, confined areas, which can be imaged quickly in their entireties. Thus, the method enables multiplexed ISH, such as multiplexed FISH.

The method has utility in theranosis, which is commonly referred to as companion diagnostics, such as for the purpose of selecting a treatment option that is more likely to be successful than another treatment option, prognostics, likely treatment responses, monitoring of prophylactic or therapeutic treatment, assessment of therapeutic efficacy, and the like.

A more specific example is companion diagnostics for targeted therapies of cancer. In distinct contrast to chemotherapy, which interferes with all rapidly dividing cells, targeted therapies (also referred to as molecularly targeted therapies) block the growth of cancer cells by interfering with specific targeted molecules needed for carcinogenesis and tumor growth. Targeted therapies are expected to be more effective and less harmful to normal cells. The main categories of targeted therapy are small molecules, small molecule drug conjugates, and monoclonal antibodies. Currently, there are targeted therapies for breast cancer, multiple myeloma, lymphoma, prostate cancer, melanoma, and other cancers.

If the patient has been diagnosed as having cancer, or is suspected of having cancer, preferably, and even desirably, the sample of cells is prepared for multiplexed ISH (e.g., multiplexed FISH) with multiple (i.e., more than one, such as two, three, four, five, six, or more) probes, wherein each probe or a combination of probes (e.g., two, three, or more), for example, enables the detection of a cancer biomarker. In this regard, the multiplexed FISH preferably, and even desirably, enables the detection of multiple (i.e., more than one, such as two, three, four, five, six, or more) cancer biomarkers. While the present disclosure is cast largely in the context of cancer, in particular the diagnosis of cancer, the prognosis of cancer, the selection of cancer therapy (including combination therapy), the assessment of the efficacy of therapy, the monitoring of therapy, the monitoring of recurrence, and the like, it is to be understood that the present disclosure has utility in the context of other diseases amenable to such assessment(s) by multiplexed ISH (e.g., multiplexed FISH).

The method also has utility in tailored prescription and tailored administration of mass-produced prophylactic and therapeutic agents as well as the tailored production and tailored administration of such agents, alone or in combination with other active agents, such as in the production and prescription of pills and polypills, which contain individualized dose levels for two or more active agents. Accordingly, the method facilitates the traditional practice of pharmaceutical compounding, i.e., preparing medicine for a specific patient in response to that patient's particular needs for dosage strength and/or formulation. The production of polypills containing two or more active agents provides an alternative to the use of mass-produced, fixed dose, combination drug products.

### Terms

The following terms are relevant to the present disclosure:
"About" refers to approximately a +/-10% variation from the stated value. It is to be understood that such a variation is always included in any given value provided herein, whether or not specific reference is made to it.

"Adenoma" is a benign tumor of glandular origin. "Adenoma," "colon adenoma," "adenoma of the colon," and "colonic adenoma" may be used herein to refer to an adenoma in the colon, which is also referred to as an adenomatous polyp. An adenomatous polyp is commonly found by colonoscopy and is removed because it has a tendency to become malignant.

"Adenocarcinoma," "colon adenocarcinoma," carcinoma of the colon," and "colonic adenocarcinoma" may be used herein to refer to a malignant growth (i.e., cancer) in the colon.

"ALK" may be used herein to refer to the anaplastic lymphoma kinase (ALK) gene, which is located on chromosome 2. The Entrez Gene and HGNC cytogenetic bands are 2p23, whereas the Ensembl cytogenetic band is 2p23.1. Aliases include anaplastic lymphoma receptor tyrosine kinase, CD246, CD246 antigen, EC 2.7.10.1, NBLST3, Ki-1, ALK tyrosine kinase receptor, and mutant anaplastic lymphoma kinase. "ALK" is also used herein to refer to a probe or a set of probes used to determine a chromosomal abnormality involving ALK. A probe for detecting a parameter involving ALK by *in situ* hybridization, such as FISH, preferably hybridizes to the p23 region of chromosome 2 (2p23), which comprises the ALK gene. Reference DNA sequences include, but are not limited to, NC_000002.11 and NT_022184.15.

"AURKA" may be used herein to refer to the aurora kinase A gene located at q13 on human chromosome 20. The Entrez Gene and HGNC cytogenetic bands are 20q13, whereas the Ensembl cytogenetic band is 20q13.2. Aliases for AURKA include ARK1, BTAK, STK15, STK6, A1K, PPP1R47, STK7, serine/threonine kinase 6, Aurora 2, Aurora-related kinase 1, Aurora/IPL1-related kinase 1, breast tumor-amplified kinase, serine/threonine-protein kinase 15, serine/threonine-protein kinase 6, serine/threonine-protein kinase aurora-A, ARK-1, AURA, hARK1, AURORA2, AurA, serine/threonine kinase 15, aurora/IPL1-like kinase, breast-tumor-amplified kinase, IPL1-related kinase, protein phosphatase 1 regulatory subunit 47, serine/threonine protein kinase 15, AIRK1, AYK1, EC2.7.11.1, and IAK1. "AURKA" is also used herein to refer to a probe or a set of probes used to determine a chromosomal abnormality involving AURKA, such as an increase in copy number. A probe for detecting a parameter involving AURKA, such as the copy number of AURKA, a copy number ratio involving AURKA, or the percentage gain of AURKA, by *in situ* hybridization, such as FISH, preferably hybridizes to the q13 region of chromosome 20 (20q13), which comprises the AURKA gene. DNA reference sequences include, but are not limited to, NC_000020.10 and NT_011362.10.

"Biomarker," as defined by the National Institutes of Health, is "a characteristic that is objectively measured and evaluated as an indicator of normal biologic processes, pathogenic processes, or pharmacologic responses to a therapeutic intervention."

"Break-apart probe" may be used herein to refer to a combination of two distinctly, detectably labeled probes, which enable the detection of a translocation. For example, one probe can hybridize at or near the 5' end of a given gene and fluoresce at one wavelength, whereas the other probe can hybridize at or near the 3' end of the given gene and fluoresce at a different wavelength. When the chromosome is in its native state, the colors combine as a single color, e.g., yellow. When the chromosome undergoes a translocation, the colors separate, e.g., red and green.

"Brush cytology specimen" is a sample of cells obtained by brushing an epithelial surface, e.g., gastrointestinal tract, lung, or cervix.

"Cancer diagnosis" generally refers to an identification of a type of cancer.

"Cancer prognosis" generally refers to a forecast or prediction of the probable course (e.g., disease progression) or outcome (e.g., metastasis or death) of the cancer.

"CDKN2" may be used herein to refer to cyclin-dependent kinase inhibitor 2A. The Entrez Gene and HGNC cytogenetic bands are 9p21, whereas the Ensembl cytogenetic band is 9p21.3. Aliases include cyclin-dependent kinase 4 inhibitor A, CDK4I, MLM, MTS1, MTS-1, CMM2, P16, ARF, INK4, INK4A, P14, P14ARF, P16-INK4A, P16INK4, P16INK4A, P19, P19ARF, TP16, CDK4 inhibitor P16-INK4, cell cycle negative regulator β, p14ARF, p16-INK4, P16-INK4a, p16INK4A, and p19ARF "CDKN2" is also used herein to refer to a probe or a set of probes used to determine a chromosomal abnormality involving CDKN2. A probe for detecting a parameter involving CDKN2 by *in situ* hybridization, such as FISH, preferably hybridizes to the p21 region of chromosome 9 (9p21), which comprises the CDKN2 gene. "CDKN2" and "P16" may be used interchangeable herein. DNA reference sequences include NC_000009.11, NT_008413.18, and NC_018920.2.

"Chromosome enumeration probe (CEP)" or "centromeric probe" is any probe that enables the number of one or more specific chromosomes in a cell to be enumerated. A chromosome enumeration probe typically recognizes and binds to a region near to (referred to as "peri-centromeric") or at the centromere of a specific chromosome, typically a repetitive DNA sequence (e.g., alpha satellite DNA). The centromere of a chromosome is typically considered to represent that chromosome, since the centromere is required for faithful segregation during cell division. Deletion or amplification of a particular chromosomal region can be differentiated from loss or gain of the whole chromosome (aneusomy), within which it normally resides, by comparing the number of signals corresponding to the particular locus (copy number) to the number of signals corresponding to the centromere. One method for making this comparison is to divide the number of signals representing the locus by the number of signals representing the centromere. Ratios of less than one indicate relative loss or deletion of the locus and ratios greater than one indicate relative gain or amplification of the locus. Similarly, comparison can be made between two different loci on the same chromosome, for example on two different arms of the chromosome, to indicate imbalanced gains or losses within the chromosome. In lieu of a centromeric probe for a chromosome, one of skill in the art will recognize that a chromosomal arm probe may alternately be used to approximate whole chromosomal loss or gain. However, such probes are not as accurate at enumerating chromosomes, since the loss of signals for such probes may not always indicate a loss of the entire chromosome. Examples of chromosome enumeration probes include CEP® probes commercially available from Abbott Molecular, Inc., Des Plaines, IL (formerly Vysis, Inc., Downers Grove, IL). Specific examples of chromosome enumeration probes or centromeric probes include probes for chromosome 1, chromosome 2, chromosome 3, chromosome 4, chromosome 5, chromosome 6, chromosome 7, chromosome 8, chromosome 9, chromosome 10, chromosome 11, chromosome 12, chromosome 13, chromosome 14, chromosome 15, chromosome 16, chromosome 17, chromosome 18, chromosome 19, chromosome 20, chromosome 21, chromosome X, and chromosome Y. Specific examples of CEP® probes include CEP1, CEP2, CEP3, CEP4, CEP5, CEP6, CEP7, CEP8, CEP9, CEP10, CEP11, CEP12, CEP13, CEP14, CEP15, CEP16, CEP17, CEP18, CEP19, CEP20, CEP21, CEPX and CEPY.

"Copy number" is a measurement of DNA, whether of a single locus, one or more loci, or an entire genome. A "copy number" of two is "wild-type" in a human (because of diploidy, except for sex chromosomes). A "copy number" of other than two in a human (except for sex chromosomes) deviates from wild-type. Such deviations include amplifications, i.e., increases in copy numbers, and deletions, i.e., decreases in copy numbers and even the absence of copy numbers.

"Core needle biopsy," "core biopsy," or "incisional biopsy" is used herein to refer to the use of a needle or an incision to remove a sample of tissue with preservation of the histological architecture of the tissue.

"DCC" may be used herein to refer to the deleted in colorectal carcinoma gene located in the region q21 on human chromosome 18. The Entrez Gene cytogenetic band is 18q21.3, whereas the Ensembl cytogenetic band is 18q21.2 and the HGNC cytogenetic band is 18q21.1. Aliases include IGDCC1, colorectal cancer suppressor, immunoglobulin superfamily DCC subclass member 1, tumor suppressor protein DCC, CRC18, CRCR1, colorectal tumor suppressor, deleted in colorectal cancer protein, and netrin receptor DCC. "DCC" also may be used herein to refer to a probe or a set of probes that can be used to determine a chromosomal abnormality involving DCC, such as DCC copy number loss. A probe for detecting a parameter involving DCC, such as the copy number of DCC, a copy number ratio involving DCC, or the percentage gain of DCC, by *in situ* hybridization, such as FISH, preferably hybridizes to the q21 region of chromosome 18 (18q21), which comprises the DCC gene. DNA reference sequences include, but are not limited to, NC_000018.9 and NT_010966.14.

"EGFR" may be used herein to refer to the epidermal growth factor receptor gene located in the region p12 on human chromosome 7. The Entrez Gene cytogenetic band and the HGNC cytogenetic band are 7p12, whereas the Ensembl cytogenetic band is 7p11.2. Aliases include avian erythroblastic leukemia viral (v-erb-b) oncogene homolog, ERBB, ERBB1, proto-oncogene c-ErbB-1, receptor tyrosine-protein kinase erbB-1, HER1, EC 2.7.10.1, PlG61, cell growth inhibiting protein 40, cell proliferation-inducing protein 61, mENA, and EC 2.7.10. "EGFR" also may be used herein to refer to a probe or a set of probes that can be used to determine a chromosomal abnormality involving EGFR, such as EGFR copy number gain. A probe for detecting a parameter involving EGFR, such as the copy number of EGFR, a copy number ratio involving EGFR, or the percentage gain of EGFR, by *in situ* hybridization, such as FISH, preferably hybridizes to the p12 region of chromosome 7 (7p12), which comprises the EGFR gene. DNA reference sequences include, but are not limited to, NC_000007.13, NT_033968.6, and NT_079592.2.

"ERG" may be used herein to refer to V-Ets avian erythroblastosis virus E26 oncogene homolog. The Entrez Gene and HGNC cytogenetic bands are 21q22.3, whereas the Ensembl genetic band is 21q22.2. Aliases include V-Ets avian erythroblastosis virus E26 oncogene related, transcriptional regulator ERG (transforming protein ERG), V-Ets erythroblastosis virus E26 oncogene like, TMPRSS2-ERG prostate cancer specific, erg-3, ets-related, p55, TMPRSS2/ERG fusion, transcriptional regulator ERG, V-Ets erythroblastosis virus E26 oncogene homolog, and transforming protein ERG. "ERG" is also used herein to refer to a probe or a set of probes used to determine a chromosomal abnormality involving ERG. A probe for detecting a parameter involving ERG by *in situ* hybridization, such as FISH, preferably hybridizes to the q22 region of chromosome 21 (21q22), which comprises the ERG gene. Reference DNA sequences include NC_000021.8, NC_018932.2, and NT_011512.11.

"Excisional biopsy" is used herein to refer to the removal of an entire lump or suspicious area.

"FGFR1" may be used herein to refer to fibroblast growth factor receptor 1. The Entrez Gene and HGNC cytogenetic bands are 8p12, whereas the Ensembl cytogenetic band is 8p11.22. Aliases include FLT2, KAL2, Fms-related tyrosine kinase 2, basic fibroblast growth factor receptor 1, Fms-like tyrosine kinase 2, proto-oncogene C-Fgr, BFGFR, CEK, FGFBR, FGFR-1, FLG, FLT-2, HBGFR, bFGF-R-1, EC 2.7.10, EC 2.7.10.1, OGD, Pfeiffer syndrome, CD331, CD331 antigen, HH2, N-SAM, FGFR1/PLAG1 fusion, heparin-binding growth factor receptor, hydroxyaryl-protein kinase, N-SAM, N-sam, FGFR1/PLAG1 fusion, heparin-binding growth factor receptor, and hydroxyaryl-protein kinase. "FGFR1" is also used herein to refer to a probe or a set of probes used to determine a chromosomal abnormality involving FGFR1. A probe for detecting a parameter involving FGFR1 by *in situ* hybridization, such as FISH, preferably hybridizes to the p11-12 regions of chromosome 8 (8p1 1-12), which comprises the FGFR1 gene. Reference DNA sequences include NC_000008.10, NT_167187.1, and NC_018919.2.

"FGFR2" may be used herein to refer to fibroblast growth factor receptor 2. The Entrez Gene cytogenetic band is 10q26, whereas the Ensembl cytogenetic band is 10q26.13, and the HGNC cytogenetic band is 10q25.3-q26. Aliases include FGFR-2, BEK, keratinocyte growth factor receptor, KGFR, CFD1, JWS, bacteria-expressed kinase, EC 2.7.10.1, craniofacial dysostosis 1, Crouzon syndrome, Jackson-Weiss syndrome, Pfeiffer syndrome, BBDS, BFR-1, CD332, CD332 antigen, CEK3, ECT1, TK14, TK25, BEK fibroblast growth factor receptor, FGF receptor, hydroxyaryl-protein kinase, protein tyrosine kinase receptor like 14, soluble FGFR4 variant 4, K-SAM, K-sam, KSAM, and EC 2.7.10. "FGFR2" is also used herein to refer to a probe or a set of probes used to determine a chromosomal abnormality involving FGFR2. A probe for detecting a parameter involving FGFR2 by *in situ* hybridization, such as FISH, preferably hybridizes to the q14 region of chromosome 10 (10q14), which comprises the FGFR2 gene. Reference DNA sequences include NC_000010.10, NC_018921.2, and NT_030059.13.

"Fine needle aspiration" or "needle aspiration biopsy" is used herein to refer to the use of a needle to remove a sample of cells from a tissue without preserving the histological architecture of the tissue from which the cells are removed. Fine needle aspiration also can be used to remove a sample a fluid, such as from a cyst.

"Fixatives" include, but are not limited to, alcohol solutions, acid acetone solutions, aldehydes (such as formaldehyde, paraformaldehyde, and glutaraldehyde), methanol/acetic acid, and formalin.

"HER2" may be used herein to refer to V-Erb-B2 avian erythroblastic leukemia viral oncogene homolog 2. The Entrez Gene and Ensembl cytogenetic bands are 17q12, whereas the HGNC cytogenetic band is 17q11.2-q12. Aliases include HER-2, HER-2/neu, TKR1, NGL, NEU, neuro/glioblastoma derived oncogene homolog, metastatic lymph node gene 19 protein, proto-oncogene C-ErbB-2, proto-oncogene Neu, tyrosine kinase-type cell surface receptor HER2, MLN 19, p185erbB2, EC 2.7.10.1, V-Erb-B2 avian erythroblastic leukemia viral oncogene homolog 2, CD340, C-Erb B2/Neu protein, herstatin, neuroblastoma/glioblastoma derived oncogene homolog, receptor tyrosine-protein kinase ErbB-2, V-Erb-B2 erythroblastic leukemia viral oncogene homolog 2, MLN19, CD340 antigen, and EC 2.7.10. "HER2" is also used herein to refer to a probe or a set of probes used to determine a chromosomal abnormality involving HER2. A probe for detecting a parameter involving HER2 by *in situ* hybridization, such as FISH, preferably hybridizes to the q11-12 region of chromosome 17 (17q11-12), which comprises the HER2 gene. Reference DNA sequences include NC_000017.10, NT_010783.15, and NC_018928.2.

"Labeled," "labeled with a detectable label," and "detectably labeled" are used interchangeably herein to indicate that an entity (e.g., a probe) can be detected. "Label" and "detectable label" mean a moiety attached to an entity to render the entity detectable, such as a moiety attached to a probe to render the probe detectable upon binding to a target sequence. The moiety, itself, may not be detectable but may become detectable upon reaction with yet another moiety. Use of the term "detectably labeled" is intended to encompass such labeling. The detectable label can be selected such that the label generates a signal, which can be measured and the intensity of which is proportional to the amount of bound entity. A wide variety of systems for labeling and/or detecting molecules, such as nucleic acids, e.g., probes, are well-known. Labeled nucleic acids can be prepared by incorporating or conjugating a label that is directly or indirectly detectable by spectroscopic, photochemical, biochemical, immunochemical, electrical, optical, chemical, or other means. Suitable detectable labels include radioisotopes, fluorophores, chromophores, chemiluminescent agents, microparticles, enzymes, magnetic particles, electron dense particles, mass labels, spin labels, haptens, and the like. Fluorophores and chemiluminescent agents are preferred herein.

"Lavage" is used herein to refer to the introduction of a fluid into a cavity of the body, such as the lung, and collection of the fluid for examination.

"Locus-specific probe" and "locus-specific identifier (LSI)" may be used interchangeably herein to refer to a probe that selectively binds to a specific locus in a region on a chromosome, e.g., a locus that has been determined to undergo gain/loss in metastasis. A probe can target coding or non-coding regions, or both, including exons, introns, and/or regulatory sequences, such as promoter sequences and the like.

"MET" may be used herein to refer to the met proto-oncogene. The Entrez Gene and HGNC cytogenetic bands are 7q31, whereas the Ensembl cytogenetic band is 7q31.2. Aliases include hepatocyte growth factor receptor, proto-oncogene C-Met, scatter factor receptor, tyrosine-protein kinase Met, HGF receptor, HGF/SF receptor, SF receptor, EC 2.7.10.1, AUTS9, HGFR, RCCP2, c-Met, Met proto-oncogene tyrosine kinase, and EC 2.7.10. "MET" is also used herein to refer to a probe or a set of probes used to determine a chromosomal abnormality involving MET. A probe for detecting a parameter involving MET by *in situ* hybridization, such as FISH, preferably hybridizes to the q31 region of chromosome 7 (7q31), which comprises the MET gene. Reference DNA sequences includes NC_000007.13, NT_007933.15, NC_018918.2, and NT_079596.2.

"MCL1" may be used herein to refer to the myeloid cell leukemia sequence 1 (BCL2-related). The Entrez Gene and HGNC cytogenetic bands are 1q21, whereas the Ensembl cytogenetic band is 1q21.3. Aliases include Bcl-2-like protein 3, Bcl-2-related protein EAT/Mcl1, BCL2L3, mcl1/EAT, EAT, MCL1-ES, MCL1L, MCL1S, Mcl-1, TM, bcl2-L-3, induced myeloid leukemia cell differentiation protein Mcl-1, myeloid cell leukemia ES, and Bcl2-L-3. "MCL1" is also used herein to refer to a probe or a set of probes used to determine a chromosomal abnormality involving MCL1. A probe for detecting a parameter involving MCL1 by *in situ* hybridization, such as FISH, preferably hybridizes to the q21 region of chromosome 1 (1q21), which comprises the MCL1 gene. Reference DNA sequences include NC_000001.10, NC_018912.2, and NT_004487.19.

"MYC" may be used herein to refer to the c-myc oncogene located in the region of q24 on chromosome 8. The Entrez Gene and Ensembl cytogenetic bands are 8q24.21, whereas the HGNC cytogenetic band is 8q24. Aliases include vHLHe39, c-Myc, v-myc avian myelocytomatosis viral oncogene homolog, Class E basic helix-loop-helix protein 39, proto-oncogene c-Myc, transcription factor p64, MRTL, avian myelocytomatosis viral oncogene homolog, myc proto-oncogene protein, myc-related translation/localization regulatory factor, and BHLHE39. MYC also may be used herein to refer to a probe or a set of probes that can be used to determine a chromosomal abnormality involving MYC, such as MYC copy number gain. A probe for detecting a parameter involving MYC, such as the copy number of MYC, a copy number ratio involving MYC, or the percentage gain of MYC, by *in situ* hybridization, such as FISH, preferably hybridizes to the q24 region of chromosome 8 (8q24), which comprises the MYC gene. DNA reference sequences include, but are not limited to, NC_000008.10 and NT_008046.16.

"NTRK1" may be used herein to refer to neurotrophic tyrosine kinase, receptor, type 1. The Ensemble cytogenetic band is 1q23.1, whereas the Entrez Gene and HGNC cytogenetic bands are 1q21-q22. Aliases include MTC, TRKA, high affinity nerve growth factor receptor, tropomyosin-related kinase A, tyrosine kinase receptor A, TRK, Trk-A, gp140trk, p140-TrkA, TRK1-transforming tyrosine kinase protein, EC 2.7.10.1, TRK1, oncogene TRK, neurotrophic tyrosine kinase receptor type 1, tyrosine kinase receptor, and EC 2.7.10. "NTRK1" is also used herein to refer to a probe or a set of probes used to determine a chromosomal abnormality involving NTRK1. A probe for detecting a parameter involving NTRK1 by *in situ* hybridization, such as FISH, preferably hybridizes to the q21-23 region of chromosome 1 (lq21-23), which comprises the NTRK1 gene. Reference DNA sequences include NC_000001.10, NT_004487.19, and NC_018912.2.

"Nucleic acid sample" refers to a sample comprising nucleic acid in a form suitable for hybridization with a probe, such as a sample comprising nuclei or nucleic acids isolated or purified from such nuclei. The nucleic acid sample may comprise total or partial (e.g., particular chromosome(s)) genomic DNA, total or partial mRNA (e.g., particular chromosome(s) or gene(s)), or selected sequence(s). Condensed chromosomes (such as are present in interphase or metaphase) are suitable for use as targets in *in situ* hybridization, such as FISH and multiplexed FISH.

"PIK3CA" may be used herein to refer to phosphatidylinositol-4,5-bisphosphate 3-kinase, catalytic subunit α. The Entrez Gene cytogenetic band is 3q26.3, whereas the Ensembl cytogenetic band is 3q26.32 and the HGNC cytogenetic band is 3q26.3. Aliases include phosphoinositide-3-kinase catalytic α polypeptide, phosphatidylinositol 4,5-bisphosphate 3-kinase catalytic subunit α isoform, phosphatidylinositol-4,5-bisphosphate 3-kinase 110 kDa catalytic subunit α, phosphatidylinositol-4-5, bisphosphate 3-kinase catalytic subunit α isoform, PI3-kinase P110 subunit α, PI3-kinae subunit α, Ptdlns-3-kinase subunit PI 10-α, Ptdlns-3-kinase subunit α, serine/threonine protein kinase PIK3CA, PI3K, PI3K-α, CLOVE, EC 2.7.1.153, EC 2.7.11.1, CWS5, PI3Kalpha, MCAP, p110α, p110-α, phosphatidylinositol 3-kinase catalytic 110-KD α, phosphatidylinositol 3-kinase catalytic α polypeptide, MCM, MCMTC, phosphatidylinositol 4,5-bisphosphate 3-kinase 110 kDa catalytic subunit α, phosphoinositide-3-kinase catalytic α polypeptide, and EC 2.7.1. "PIK3CA" is also used herein to refer to a probe or a set of probes used to determine a chromosomal abnormality involving PIK3CA. A probe for detecting a parameter involving PIK3CA by *in situ* hybridization, such as FISH, preferably hybridizes to the q26 region of chromosome 3 (3q26), which comprises the PIK3CA gene. Reference DNA sequences include NC_000003.11, NC_018914.2, and NT_005612.16.

"Predetermined cutoff' and "predetermined level" refer generally to a cutoff value that is used to assess diagnostic/prognostic/therapeutic efficacy results by comparing the assay results against the predetermined cutoff/level, where the predetermined cutoff/level already has been linked or associated with various clinical parameters (e.g., severity of disease, progression/nonprogression/improvement, etc.).

"Probe," in the context of the present disclosure, is an oligonucleotide or polynucleotide that can selectively hybridize to at least a portion of a target sequence under conditions that allow for or promote selective hybridization. In general, a probe can be complementary to the coding or sense (+) strand of DNA or complementary to the non-coding or anti-sense (-) strand of DNA (sometimes referred to as "reverse-complementary"). Probes can vary significantly in length. A length of about 10 to about 100 nucleotides, such as about 15 to about 75 nucleotides, e.g., about 15 to about 50 nucleotides, can be preferred in some applications, whereas a length of about 50-1x10⁵ nucleotides can be preferred for chromosomal probes and a length of about 25,000 to about 800,000 nucleotides can be preferred for locus-specific probes. Probe also may be used herein to refer to a detectably labeled antibody or antigen-binding fragment thereof, for example, in the context of IHC and the confirmation of cells as epithelial cells.

"Prognosis" is a prediction of the probable course and/or outcome of a disease, such as the likelihood of recovery. Thus, prognosis encompasses an improvement in disease status, a worsening in disease status, and no change in disease status.

"PTEN" may be used herein to refer to phosphatase and tensin homolog. The Entrez Gene cytogenetic band is 10q23.3, whereas the Ensembl cytogenetic band is 10q23.31, and the HGNC cytogenetic band is 10q23. Aliases include phosphatase and tensin-like protein, phosphatidylinositol 3,4,5-trisphosphate 3-phosphatase and dual-specificity protein phosphatase, PTEN1, mutated in multiple advanced cancers 1, MMAC1, MMAC1 phosphatase and tensin homolog deleted on chromosome 10, BZS, MHAM, TEP1, GLM2, 10q23del, CWS1, DEC, EC 3.1.3.16, EC 3.1.3.48, and EC 3.1.3.67. "PTEN" is also used herein to refer to a probe or a set of probes used to determine a chromosomal abnormality involving PTEN. A probe for detecting a parameter involving PTEN by *in situ* hybridization, such as FISH, preferably hybridizes to the q23 region of chromosome 10 (10q23), which comprises the PTEN gene. Reference DNA sequences include NC_000010.10, NC_018921.2, and NT 030059.13.

"RB1" may be used herein to refer to the retinoblastoma gene located in the region of q14 on human chromosome 13. The Entrez, Ensembl, and HGNC cytogenetic bands are 13q14.2. Aliases include OSRC, RB, p105-Rb, pRb, pp110, osteosarcoma, retinoblastoma suspectibility protein, retinoblastoma-associated protein, and Rb. "RB1" may be used interchangeably herein with "13q14". "RB1" or "13q14" also may be used herein to refer to a probe or a set of probes that can be used to determine a chromosomal abnormality involving RBI or 13ql4, such as RB1 or 13q14 copy number gain. A probe for detecting a parameter involving RBI or 13ql4, such as the copy number of RB1 or 13q14, a copy number ratio involving RBI or 13q14, or the percentage gain of RBI or 13q14, by *in situ* hybridization, such as FISH, preferably hybridizes to the q14 region of chromosome 13 (13q14), which comprises the RB1 gene. DNA reference sequences include, but are not limited to, NC_000013.10 and NT_024524.14.

"RET" may be used herein to refer to the ret proto-oncogene is located on chromosome 10. The Entrez Gene and HGNC cytogenetic bands are 10q11.2, whereas the Ensembl cytogenetic band is 10q11.21. Aliases include CDHF12, CDHR16, PTC, RET51, HSCR1, MEN2A, MEN2B, MTC1, EC 2.7.10.1, EC 2.7.10, cadherin family member 12, proto-oncogene c-Ret, Hirschsprung disease 1, multiple endocrine neoplasia and medullary thyroid carcinoma 1, RET-ELE1, cadherin-related family member 16, hydroxyaryl-protein kinase, proto-oncogene tyrosine-protein kinase receptor Ret, receptor tyrosine kinase, and RET transforming sequence. "RET" is also used herein to refer to a probe or a set of probes used to determine a chromosomal abnormality involving RET. A probe for detecting a parameter involving RET by *in situ* hybridization, such as FISH, preferably hybridizes to the q11 region of chromosome 10 (10q11), which comprises the RET gene. Reference DNA sequences include, but are not limited to, NC_000010.10 and NT_033985.7.

"Risk of progression" is used herein to refer to a worsening in disease status.

"ROS" is used herein to refer to the C-Ros oncogene 1, receptor tyrosine kinase. The Entrez Gene cytogenetic band is 6q22, whereas the Ensembl cytogenetic band is 6q22.1 and the HGNC cytogenetic band is 6q21-q22. Aliases include MCF3, V-Ros UR2 sarcoma virus oncogene homolog 1, V-Ros avian UR2 sarcoma virus oncogene homolog 1, V-Ros UR2 sarcoma virus oncogene homolog 1 (avian), c-ros-1, proto-oncogene tyrosine-protein kinase, transmembrane tyrosine-specific protein kinase, proto-oncogene C-Ros, proto-oncogene C-Ros-1, receptor tyrosine kinase C-Ros oncogene, C-Ros receptor tyrosine kinase, and EC 2.7.10.1. "ROS" is also used herein to refer to a probe or a set of probes used to determine a chromosomal abnormality involving ROS. A probe for detecting a parameter involving ROS by *in situ* hybridization, such as FISH, preferably hybridizes to the q22 region of chromosome 6 (6q22), which comprises the ROS gene. Reference DNA sequences include NC_000006.11, NC_018917.2, and NT_025741.15.

"Section" of a tissue sample is a single part or piece of a tissue sample, e.g., a thin slice of tissue or cells cut from a tissue sample. Two or more sections of tissue samples may be taken and analyzed. If desired, a single section can be analyzed at various levels, e.g., morphological and molecular (e.g., nucleic acid and protein).

"Selectively hybridize to" (as well as "selective hybridization," "specifically hybridize to," and "specific hybridization"), in the context of the present disclosure, refers to the binding, duplexing, or hybridizing of a nucleic acid molecule preferentially to a particular nucleotide sequence under stringent conditions. The term "stringent conditions" refers to conditions under which a probe will hybridize preferentially to its target sequence, and to a lesser extent to, or not at all to, other non-target sequences. A "stringent hybridization" and "stringent hybridization wash conditions" in the context of nucleic acid hybridization (e.g., as in array, Southern hybridization, Northern hybridization, or FISH) are sequence-dependent, and differ under different conditions. An extensive guide to the hybridization of nucleic acids is found in, e.g., Tijssen, Laboratory Techniques in Biochemistry and Molecular Biology--Hybridization with Nucleic Acid Probes, Part I, Ch. 2, "Overview of principles of hybridization and the strategy of nucleic acid probe assays," Elsevier, NY (1993). Generally, highly stringent hybridization and wash conditions are selected to be about 5 °C lower than the thermal melting point (Tₘ) for the specific sequence at a defined ionic strength and pH. The Tₘ is the temperature (under defined ionic strength and pH) at which 50% of the target sequence hybridizes to a perfectly matched probe. Very stringent conditions are selected to be equal to the Tₘ for a particular probe. An example of stringent hybridization conditions for hybridization of complementary nucleic acids, which have more than 100 complementary residues, on an array or on a filter in a Southern or Northern blot is 42 °C using standard hybridization solutions (see, e.g., Sambrook and Russell, Molecular Cloning: A Laboratory Manual, 3rd ed., Vol. 1-3, Cold Spring Harbor Laboratory, Cold Spring Harbor Press, NY (2001)).

"STAT 1" may be used herein to refer to the signal transducer and activator of transcription 1 gene located at q32 on human chromosome 2. The Entrez Gene and Ensembl cytogenetic bands are 2q32.3, whereas the HGNC cytogenetic band is 2q32.2-q32.3. Aliases for STAT1 include STAT-1, ISGF-3, STAT91, transcription factor ISGF-3 components p91/p84, CANDF7, and signal transducer and activator of transcription 1-α/β. "STAT1" is also used herein to refer to a probe or a set of probes used to determine a chromosomal abnormality involving STAT1, such as an increase in copy number. A probe for detecting a parameter involving STAT1, such as the copy number of STAT1, a copy number ratio involving STAT1, or the percentage gain of STAT1, by *in situ* hybridization, such as FISH, preferably hybridizes to the q32 region of chromosome 2 (2q32), which comprises the STAT1 gene. DNA reference sequences include, but are not limited to, NC_000002.11 and NT_005403.17.

"Target sequence," "target region," and "nucleic acid target" refer to a nucleotide sequence that resides at a specific chromosomal location whose loss and/or gain, for example, is being determined.

"TERC" may be used herein to refer to telomerase RNA component. The Ensembl and HGNC cytogenetic bands are 3q26.2, whereas the Entrez Gene band is 3q26. Aliases include TR, TRC3, small Cajal body-specific RNA 19, DKCA1, PFBMFT2, SCARNA19, and hTR. "TERC" is also used herein to refer to a probe or a set of probes used to determine a chromosomal abnormality involving TERC. A probe for detecting a parameter involving TERC by *in situ* hybridization, such as FISH, preferably hybridizes to the q26 region of chromosome 3 (3q26), which comprises the TERC gene. Reference DNA sequences include NC_000003.11, NC_018914.2, and NT_005612.16.

"Theranosis" is used herein to refer to processes, such as diagnostic tests, in particular diagnostic tests employing ISH, such as FISH, used to tailor therapy for a patient. Through theranosis the suitability of a drug for treatment of a particular patient can be assessed. In addition, the efficacy of treatment of the patient with the drug can be assessed.

"ZNF217" may be used herein to refer to zinc finger protein 217. The Entrez Gene, Ensembl and HGNC cytogenetic bands are 20q13.2. An alias is ZABC1. "ZNF217" is also used herein to refer to a probe or a set of probes used to determine a chromosomal abnormality involving ZNF217. A probe for detecting a parameter involving ZNF217 by *in situ* hybridization, such as FISH, preferably hybridizes to the q13 region of chromosome 20 (20q13), which comprises the ZNF217 gene. Reference DNA sequences include NC_000020.10, NT_011362.10, and NC_018931.2.

The terminology used herein is for the purpose of describing particular embodiments only and is not otherwise intended to be limiting.

### Method of Preparation of Cells

A method of preparing a sample of cells is provided. The sample of cells can be prepared from any suitable patient specimen comprising a tissue or a clump of cells. Typically, patient specimens are obtained by methods such as fine needle aspiration (FNA), core needle biopsy, excisional biopsy, resection, lavage (e.g., bronchial lavage), brushing, and the like. Also typically, the patient specimen can be fixed and embedded, fixed but not embedded, partially fixed (e.g., in a preservative such as PreservCyt®, which is a methanol-water, buffered preservative solution manufactured by Hologic Inc., Marlborough, MA), frozen (e.g., freshly frozen), or fresh. Preferably, the patient specimen is obtained by FNA, core needle biopsy, excisional biopsy, or resection. A tissue specimen or specimen comprising a clump (i.e., an aggregate or aggregation) of cells also can be obtained by lavage and brushing in some instances. Preferably, the patient specimen is not embedded and is either (a) fresh, (b) thawed, freshly frozen, or (c) partially fixed inasmuch as it is an object of the present disclosure to obtain results of hybridization with a probe, such as ISH (e.g., FISH), in particular multiplexed ISH (e.g., multiplexed FISH), or binding with an antibody (as in IHC), as quickly as possible after the specimen is obtained from the patient, such as in less than about 14 hours. In view of the foregoing, it is preferable, and even desirable, to prepare a sample of cells from the patient specimen as quickly as possible (e.g., immediately) after the specimen is obtained from the patient. As indicated above, by avoiding the use of embedded specimens, truncation artifacts associated with sectioning of the embedded specimens can be avoided.

The method comprises disaggregating a patient specimen comprising a tissue or a clump of cells. The tissue or clump of cells can be disaggregated by any suitable method. Preferably, the method involves the use of centrifugation or a cell strainer.

In an embodiment, the method comprises rinsing the patient specimen, which can be a fresh specimen, a thawed, freshly frozen specimen, or a partially fixed specimen. Preferably, the specimen is rinsed at ambient temperature (e.g., room temperature) in a balanced salt solution, the composition of which maintains pH and osmotic balance and provides water and essential inorganic ions (e.g., sodium, potassium, calcium, magnesium, and chloride), at ambient temperature. Examples of balanced salt solutions include, but are not limited to, Hank's balanced salt solution (available from Life Technologies™ (Carlsbad, CA)), Alsever's solution, Earle's balanced salt solution, Gey's balanced salt solution, Dulbecco's phosphate-buffered saline, Puck's balanced salt solution, Ringer's balanced salt solution, Simm's balanced salt solution, TRIS-buffered saline, and Tyrode's balanced salt solution. A preferred balanced salt solution is Hank's balanced salt solution. After rinsing, the specimen is soaked in a balanced salt solution, such as Hank's balanced salt solution, comprising a reducing agent, such as dithiothreitol (DTT), and a chelating agent, such as ethylenediaminetetraacetic acid (EDTA). Preferably, the balanced salt solution, which comprises the reducing agent and the chelating agent and in which the specimen is soaked, is pre-warmed. After rinsing and soaking, the specimen is transferred to fresh balanced salt solution, such as Hank's balanced salt solution comprising a reducing agent, such as DTT, a source of calcium ions, such as calcium chloride, and a polar aprotic solvent, such as dimethylsulfoxide (DMSO), and mixed briefly, such as for about 5 seconds to about 10 seconds.

At this point, the specimen is disaggregated, e.g., centrifuged or strained. If centrifuged, the specimen is spun at low speed, such as 1000 x g, to pellet disaggregated cells. Disaggregated cells will pellet within a matter of minutes, such as less than about 15 minutes, such as in about 10 minutes. After centrifugation, the supernatant is discarded, and the pelleted cells are re-suspended in residual supernatant. The re-suspended cells are placed in a fixative solution, such as an aqueous solution comprising a buffer, a preservative, and either an alcohol, e.g., methanol or ethanol, PreserCyt®, or the like. If strained, the specimen is strained through a cell strainer, such as one available from Becton Dickinson, Franklin Lakes, NJ, and the strained cells are placed in a fixative solution, such as an aqueous solution comprising a buffer, a preservative, and either an alcohol, e.g., methanol or ethanol, PreservCyt®, or the like. The re-suspended cells are allowed to stand in the fixative solution for a period of time, such as a matter of minutes (e.g., about 15 minutes, about 30 minutes, or about 45 minutes) up to about an hour.

At this point, the re-suspended cells can be applied to the surface of a cell support. Any suitable cell support can be used. Examples of suitable cell supports include, but are not limited to, a cytology slide, a cyto-spin, a Tethis slide, and a microfluidic camera. The cell support can comprise two or more, such as multiple (e.g., three, four, five, six or more), discrete areas, which are amenable to nucleic acid hybridization and visualization. Examples of discrete areas include concavities, such as depressions, wells, chambers, and the like. Distribution of the re-suspended cells to two or more, such as multiple, discrete areas enables multiplex hybridization with a probe, such as ISH, e.g., multiplex FISH, and/or binding with an antibody (such as in IHC) to be performed on the cell support. Hybridization results can be visualized manually (provided fluorophores used in labeling are not far-red fluorophores, which are invisible to the human eye) or using any suitable imaging system. Examples of commercially available imaging systems include, but are not limited to, those imaging systems available from Bioview, Inc. (Billerica, MA), and ASI.

Thus, in view of the above, a method of preparing cells from a patient specimen comprising a tissue or a clump of cells is provided. The method comprises:
(i) soaking the patient specimen in a pre-warmed, balanced salt solution comprising a reducing agent and a chelating agent;
(ii) transferring the patient specimen to a fresh, balanced salt solution comprising a reducing agent, a source of calcium ions, and a polar aprotic solvent and mixing;
(iii) removing the patient specimen from the fresh, balanced salt solution and either (a) centrifuging the solution to pellet disaggregated cells or (b) straining the solution to collect disaggregated cells; and
(iv) after (iii)(a), discarding the supernatant, re-suspending the pelleted cells in residual supernatant, and placing the re-suspended cells in a fixative or, after (iii)(b), placing the strained cells in a fixative. The patient specimen can be (a) a fresh specimen, (b) a thawed, freshly frozen specimen, or (c) a partially fixed specimen.
The method can further comprise rinsing the patient specimen in a balanced salt solution prior to (i).

In a preferred embodiment, the method comprises rinsing the patient specimen, such as a sample of adenoma, in Hank's buffer at room temperature, soaking the patient specimen in pre-warmed Hank's buffer containing 20 mM DTT and 5 mM EDTA at 37 °C for around five minutes, transferring the rinsed and soaked patient specimen to fresh Hank's buffer containing 20 mM DTT, 5 mM CaCl₂, and 10% DMSO, and mixing for about 5 seconds to about 10 seconds, removing the patient specimen from the fresh Hank's buffer and either (a) centrifuging the solution at about 1000 x g for about 10 minutes to pellet disaggregated cells or (b) straining the solution to collect disaggregated cells, and either, after centrifuging, discarding the supernatant, re-suspending the pelleted cells in residual supernatant, and placing the re-suspended cells in a solution or, after straining, placing the strained cells in a solution, wherein the solution comprises from about 30 wt.% to about 60 wt.% methanol, from about 40 wt.% to about 70 wt.% water, a buffer, and a preservative and wherein the solution is kept at room temperature overnight.

The method can further comprise (v) applying the cells to a cell support. If the cell support is a cytology slide, the method can further comprise (vi) soaking the slide in a water-alcohol solution, (vii) soaking the slide in a fixative, and (viii) drying the slide. The water-alcohol solution can be 95% ethanol, and the slide can be soaked in the ethanol for about 15 minutes at room temperature. The fixative can be Carnoy's solution, and the slide can be soaked in the Carnoy's solution for about 30 minutes at room temperature. The slide can be dried at room temperature.

### Cell Support

Also provided is a cell support, such as a cytology slide, a cyto-spin, a Tethis slide, and a microfluidic camera, comprising cells prepared from a patient specimen, such as cells isolated from a sample of an adenoma, in accordance with the above methods. The cells can be applied to the cell support manually or by use of an instrument, such as the ThinPrep T-2000 processor (Hologic, Inc., Marlborough, MA) or another commercially available instrument. The cell support can comprise two or more, such as multiple (e.g., three, four, five, six or more), discrete areas, which are amenable to ISH (or IHC) and visualization, for application of cells prepared from a patient specimen in accordance with the above methods. Examples of discrete areas include concavities, such as depressions, wells, chambers, and the like. In a preferred embodiment, the cell support comprises at least six discrete areas. Preferably, and even desirably, the cells in each discrete area are contacted with at least one detectably labeled probe and, if an area is contacted with two or more detectably labeled probes, the detectably labeled probes can be distinguished as different probes. Preferably, the dimensions of the discrete areas are such that each discrete area can be viewed with an imaging system in its entirety. Also preferably, there is distance between the discrete areas, and the distance is such that a signal produced by a detectable label, such as a fluorescent label, in one discrete area does not interfere with the visualizations of a signal produced by a detectable label, such as a fluorescent label, in one or more adjacent discrete areas. In this regard, detectable labels can be selected such that detectable labels in adjacent areas emit signals or fluoresce at different wavelengths, the visualization of which do not interfere with each other. The distance between discrete areas should not be so great as to render the cell support cumbersome to use and/or incompatible with an imaging system, if one is to be used.

Cells can be applied to the surface of a cell support, such as a cytology slide, and the cell support can be soaked in an alcohol-water solution, such as 95% ethanol, for a few minutes, such as about 15 minutes, at ambient (i.e., room) temperature. Afterwards, the cell support can be soaked in an above-described fixative, such as Carnoy's solution, which is 3:1 methanol:acetic acid, for about 30 minutes at ambient (i.e., room) temperature, and dried, such as at room temperature.

The cell support, such as a slide, can be, if desired, stored at -20°C until use. Preferably, and even desirably, however, the cell support is used immediately, since it is an object of the present disclosure to obtain results of ISH (e.g., FISH), in particular multiplexed ISH (e.g., multiplexed FISH), as quickly as possible after the specimen is obtained from the patient, such as in less than about 14 hours.

Thus, in view of the above, a cell support on the surface of which are cells, which have been contacted with detectably labeled probes in accordance with the method described herein, is provided. The cell support comprises at least two discrete areas and the cells have been applied to the discrete areas, in which case each area is contacted with at least one detectably labeled probe and, if an area is contacted with two or more detectably labeled probes, the detectably labeled probes can be distinguished. For example, each discrete area can be separately contacted with a mixture of detectably labeled probes, wherein the mixture comprises:
--a two-color break-apart probe for ALK and a two-color break-apart probe for ROSI,
--a two-color break-apart probe for ALK, a two-color break-apart probe for ROS1, and a probe for c-MET,
--a two-color break-apart probe for ALK, a two-color break-apart probe for ROS1, and a two-color break-apart probe for RET,
--a two-color break-apart probe for RET and a two-color break-apart probe for NTRK1,
--a two-color break-apart probe for RET and a probe for KIF5B,
--a probe for MET, a probe for EGFR, and a CEP for chromosome 7,
--a probe for AURKA, a locus-specific probe for 9p21, a CEP for chromosome 3, a CEP for chromosome 6, and a CEP for chromosome 7,
--a probe for MYC, a probe for EGFR, a probe for DCC, a locus-specific probe for 13q14, and a CEP for chromosome 18,
--a probe for MYC, a probe for TERC, and a CEP for chromosome 7,
--a probe for MYC, a probe for HER2, a probe for ZNF217, and a locus-specific probe for 9p21,
--a probe for MCL1, a probe for FGFR2, a probe for MET, a probe for EGFR, and a CEP for chromosome 7,
--a probe for FGFR2, a probe for PIK3CA, a CEP for chromosome 3, and a CEP for chromosome 10,
--a probe for PTEN, a probe for ERG, and a CEP for chromosome 10,
--a probe for FGFR1, a probe for FGFR2, a two-color break-away probe for FGFR2, a CEP for chromosome 8, and a CEP for chromosome 10, or
--a probe for MET, a probe for PIK3CA, a probe for EGFR, a CEP for chromosome 7, and a CEP for chromosome 3.

### In situ Hybridization and Immunohistochemistry

A method of performing *in situ* hybridization (ISH) or immunohistochemistry (IHC) is provided. The method comprises contacting cells, which have been prepared in accordance with an above-described method, with a detectably labeled probe under hybridizing conditions and visualizing the detectable label in less than about 14 hours, e.g., 13.5 hours, of removal of the specimen from the patient. In an embodiment, the method comprises contacting cells, which have been disaggregated from a specimen selected from the group consisting of a fine needle aspiration, a core needle biopsy, an excisional biopsy, and a resection, with a detectably labeled probe under hybridizing conditions and visualizing the detectable label in less than about 14 hours, e.g., 13.5 hours, of removal of the specimen from the patient. The detectably labeled probe is a detectably labeled nucleic acid probe, such as a fluorescently labeled nucleic acid probe, or a detectably labeled antibody, such as a fluorescently labeled antibody (or antigenically reactive fragment thereof) probe. With regard to the above methods, hybridization and visualization can take place in substantially less than about 14 hours, such as about 6.5 hours, for example, when an oligo-based probe is used.

Preferably, the cells have been applied to a cell support. Preferably, the cell support comprises at least two discrete areas and the cells have been applied to the discrete areas, in which case each area is contacted with at least one detectably labeled probe and, if an area is contacted with two or more detectably labeled probes, the detectably labeled probes can be distinguished. The discrete areas can be separately contacted with any suitable detectably labeled probe. For example, each discrete area can be separately contacted with a mixture of detectably labeled probes, wherein the mixture comprises:
--a two-color break-apart probe for ALK and a two-color break-apart probe for ROSI,
--a two-color break-apart probe for ALK, a two-color break-apart probe for ROS1, and a probe for c-MET,
--a two-color break-apart probe for ALK, a two-color break-apart probe for ROSI, and a two-color break-apart probe for RET,
--a two-color break-apart probe for RET and a two-color break-apart probe for NTRK1,
--a two-color break-apart probe for RET and a probe for KIF5B,
--a probe for MET, a probe for EGFR, and a CEP for chromosome 7,
--a probe for AURKA, a locus-specific probe for 9p21, a CEP for chromosome 3, a CEP for chromosome 6, and a CEP for chromosome 7,
--a probe for MYC, a probe for EGFR, a probe for DCC, a locus-specific probe for 13q14, and a CEP for chromosome 18,
--a probe for MYC, a probe for TERC, and a CEP for chromosome 7,
--a probe for MYC, a probe for HER2, a probe for ZNF217, and a locus-specific probe for 9p21,
--a probe for MCL1, a probe for FGFR2, a probe for MET, a probe for EGFR, and a CEP for chromosome 7,
--a probe for FGFR2, a probe for PIK3CA, a CEP for chromosome 3, and a CEP for chromosome 10,
--a probe for PTEN, a probe for ERG, and a CEP for chromosome 10,
--a probe for FGFR1, a probe for FGFR2, a two-color break-away probe for FGFR2, a CEP for chromosome 8, and a CEP for chromosome 10, or
--a probe for MET, a probe for PIK3CA, a probe for EGFR, a CEP for chromosome 7, and a CEP for chromosome 3.

The above method can be carried out using any suitable detection method known in the art. When two or more probes are used simultaneously or sequentially on the same sample, preferably each probe is detectably labeled with a distinct label, such as a distinct fluorophore.

When the above methods are carried out by *in situ* hybridization, in which each probe is detectably labeled (and, when two or more probes are used simultaneously or sequentially on the same sample, distinctly labeled), such as by FISH, in which each probe is labeled (and, when two or more probes are used simultaneously or sequentially on the same sample, distinctly labeled) with a fluorophore, the methods can be carried out with cells (such as epithelial cells) prepared from a patient specimen (such as an adenoma) in accordance with the methods described herein. The patient specimen can be (a) fresh (fresh cells can be cultured for 1-3 days and a blocker, such as Colcemid, can be added to the culture to block the cells in metaphase, during which chromosomes are highly condensed and can be visualized), (b) thawed, freshly frozen, or (c) partially fixed (e.g., fixed in methanol, ethanol, or PreservCyt®), treated (e.g., with RNase and pepsin) to increase accessibility of target nucleic acid (e.g., DNA) and reduce non-specific binding, and then subjected to hybridization with one or more probes, washing to remove any unbound probes, and detection of hybridized probes.

Cell supports, such as cytology slides, can be prepared using a ThinPrep T-2000 processor (Hologic, Inc., Bedford, MA) in accordance with manufacturer's instructions. Slides can be placed in 2xSSC at 73 °C for two minutes, incubated with pepsin (0.5 mg/mL) at 37 °C for 10 minutes, placed in 1xPBS (phosphate-buffered saline) at room temperature for five minutes, 1% NBF (neutral-buffered formalin) at room temperature for five minutes, and 1xPBS at room temperature for five minutes. Slides can be dehydrated by placement in 70% ethanol, 85% ethanol, and 100% ethanol for one minute each and then air-dried. Probe and a coverslip can be applied, the coverslip sealed, and the slide co-denatured in ThermoBrite for two minutes at 72 °C and hybridized for up to 8 hours at 37 °C. Upon completion of hybridization, slides then can be treated with 0.4 x SSC/0.3% NP-40 for two minutes at 73 °C and 2xSSC/0.1% NP-40 for one minute at room temperature and then air-dried, mounted with 4'6'-diamidino-2-phenylindole dihydrochloride hydrate (DAPI) I anti-fade solution (Abbott Molecular, Inc., Des Plaines, IL), and covered with a coverslip.

Hybridization with one or more probes as described above using an above-described cell support can be carried out at 37 °C for up to eight hours in an automated co-denaturation instrument (HYBrite or ThermoBrite Denaturation/Hybridization System, Abbot Molecular, Inc., Des Plaines, IL) according to the manufacturer's instructions (such methods typically involve denaturation of probes and target nucleic acids). After hybridization, the cell support, e.g., cytology slide, can be placed in washing buffer (2 x saline sodium citrate/0.3% NP40; available from Abbott Molecular, Inc.) at room temperature for 2-10 minutes to remove the coverslip and then immersed in 73 °C washing buffer for two minutes, dried, and mounted with DAPI I anti-fade solution (Abbott Molecular, Inc.). Preferably, the cell support, e.g., cytology slide, is analyzed with an epi-fluorescence microscope equipped with single band-pass filters (Abbott Molecular, Inc.).

Prior to detection, cell samples may be optionally pre-selected based on apparent cytologic abnormalities. Pre-selection identifies suspicious cells, thereby allowing the screening to be focused on those cells. Pre-selection allows for faster screening and increases the likelihood that a positive result will not be missed. Cells from a biological sample can be placed on a microscope slide and visually scanned for cytologic abnormalities commonly associated with dysplastic and neoplastic cells. Such abnormalities include abnormalities in nuclear size, nuclear shape, and nuclear staining, as assessed by counterstaining nuclei with nucleic acid stains or dyes, such as propidium iodide or DAPI I, usually following hybridization of probes to their target DNAs. Typically, neoplastic cells harbor nuclei that are enlarged, irregular in shape, and/or show a mottled staining pattern. Propidium iodide, typically used at a concentration of about 0.4 µg/ml to about 5 µg/ml, is a red-fluorescing DNA-specific dye that can be observed at an emission peak wavelength of 614 nm. DAPI, typically used at a concentration of about 125 ng/ml to about 1,000 ng/ml, is a blue fluorescing DNA-specific stain that can be observed at an emission peak wavelength of 452 nm with a DAPI filter at low magnification. In this case, only those cells pre-selected for detection are subjected to counting for chromosomal losses and/or gains. Preferably, pre-selected cells on the order of at least 100, and preferably more when there appear to be many cells with abnormal nucleic using the DAPI filter, are chosen for assessing chromosomal losses and/or gains.

Alternatively, an area in a tissue evidencing some level of dysplasia or a suspicious lesion can be localized using the DAPI filter at low magnification and thoroughly inspected for the presence of nuclei harboring abnormal copy numbers of any probe. In a normal cell, two copies of a given probe will be detected. In an abnormal cell, more or less copies of a given probe will be detected. Areas with the most significant copy number changes are preferably selected for enumeration. Wherever possible, numerous abnormal areas are selected and, within each abnormal area, at least about 10 random nuclei are analyzed under high power (64 x or 100 x objective) so that at least about 100 nuclei are analyzed. Preferably, nuclei are non-overlapping and harbor sufficiently bright signals.

Alternatively, cells for detection may be chosen independent of cytologic or histologic features. For example, all non-overlapping cells in a given area or areas on a microscope slide may be assessed for chromosomal losses and/or gains. As a further example, cells on the slide, e.g., cells that show altered morphology, on the order of at least about 50, and more preferably at least about 100, in number that appear in consecutive order on a microscope slide may be chosen for assessing chromosomal losses and/or gains.

The copies of genes/loci/chromosomes are counted. If desired, the copy number can be compared to the expected or "normal" number of copies (i.e., 2 copies), wherein a copy number greater than 2 (i.e., for a gain) and a copy number less than 2 (i.e., for a loss) and/or relative loss compared based on a comparison of the copy number of an LSI probe and the copy number of a centromeric probe (also referred to herein as a chromosome enumeration probe (CEP)), as appropriate, indicates that the cell is abnormal by FISH. The presence of more than two copies of two or more loci is indicative of polysomy. An increase in copy number of a single locus, such as more than two copies of a single locus, is indicative of a single locus gain. While deparaffinization, pretreatment, staining, and routine slide washing also can be conducted in accordance with methods known in the art, use of an automated system, however, such as the VP 2000 Processor (Abbott Molecular, Inc., Des Plaines, IL), decreases the amount of time needed to prepare slides for evaluation. Slides can be prepared in large batches (e.g., 50 slides), as opposed to small batches (e.g., 4 slides) when standard Coplin jars are used for post-hybridization washing. In addition, the scoring of slides can be fully automated using automated imaging, thereby reducing the amount of hands-on time required for specimen analysis. Full automation also enables the use of an imaging algorithm that captures more abnormal cells more frequently and consistently. Also, while any suitable method of slide preparation known in the art can be used, slides are preferably prepared using ThinPrep 2000 (Hologic, Inc., Bedford, MA), which generates more uniform and consistent monolayers of cells.

Denaturation of nucleic acid targets for analysis by *in situ* hybridization and similar methods typically is done in such a manner as to preserve cell morphology. For example, chromosomal DNA can be denatured by high pH, heat (e.g., temperatures from about 70-95 °C), organic solvents (e.g., formamide), and combinations thereof. Probes, on the other hand, can be denatured by heat in a matter of minutes.

After denaturation, hybridization is carried out. Conditions for specifically hybridizing the probes to their nucleic acid targets generally include the combinations of conditions that are employable in a given hybridization procedure to produce specific hybrids, the conditions of which may easily be determined by one of ordinary skill in the art. Such conditions typically involve controlled temperature, liquid phase, and contact between a probe and a target. Hybridization conditions vary depending upon many factors including probe concentration, target length, target and probe G-C content, solvent composition, temperature, and duration of incubation. At least one denaturation step can precede contact of the probes with the targets. Alternatively, the probe and the target can be subjected to denaturing conditions together while in contact with one another, or with subsequent contact of the probe with the biological sample. Hybridization can be achieved with subsequent incubation of the probe/sample in, for example, a liquid phase containing about 2-4 x SSC and about 10-50% formamide, at a temperature in the range of about 25 to about 55° C for a time that is illustratively in the range of about 0.5 to about 96 hours. In order to increase specificity, a blocking agent, such as unlabeled blocking nucleic acid, as described in U.S. Pat. No. 5,756,696 (the contents of which are herein incorporated by reference in their entirety, and specifically for the description of the use of blocking nucleic acid), can be used. Other conditions can be readily employed for specifically hybridizing the probes to their nucleic acid targets present in the sample, as would be readily apparent to one of skill in the art. Hybridization protocols are described, for example, in Pinkel et al., PNAS USA 85: 9138-9142 (1988); In situ Hybridization Protocols, Methods in Molecular Biology, Vol. 33, Choo, ed., Humana Press, Totowa, NJ (1994); and Kallioniemi et al., PNAS USA 89: 5321-5325 (1992).

Upon completion of a suitable incubation period, non-specific binding of chromosomal probes to sample DNA can be removed by a series of washes. Temperature and salt concentrations are suitably chosen for a desired stringency. The level of stringency required depends on the complexity of a specific probe sequence in relation to the genomic sequence, and can be determined by systematically hybridizing probes to samples of known genetic composition. In general, high stringency washes can be carried out at a temperature in the range of about 65 to about 80° C with about 0.2 x to about 2 x SSC and about 0.1% to about 1% of a non-ionic detergent, such as Nonidet P-40 (NP40). If lower stringency washes are required, the washes can be carried out at a lower temperature with an increased concentration of salt.

When fluorophore-labeled probes or probe compositions are used, the detection method can involve fluorescence microscopy, flow cytometry, or other means for determining probe hybridization. Any suitable microscopic imaging method can be used in conjunction with the methods described herein for observing multiple fluorophores. In the case where fluorescence microscopy is employed, hybridized samples can be viewed under light suitable for excitation of each fluorophore and with the use of an appropriate filter or filters. Automated digital imaging systems, such as the MetaSystems, BioView or Applied Imaging systems, alternatively can be used, along with signal enumeration and data acquisition algorithms.

Depending on the method employed, a digital image analysis system can be used to facilitate the display of results and to improve the sensitivity of detecting small differences in fluorescence intensity. An exemplary system is QUIPS (an acronym for quantitative image processing system), which is an automated image analysis system based on a standard fluorescence microscope equipped with an automated stage, focus control and filter wheel (Ludl Electronic Products, Ltd., Hawthorne, NY). The filter wheel is mounted in the fluorescence excitation path of the microscope for selection of the excitation wavelength. Special filters (Chroma Technology, Brattleboro, VT) in the dichroic block allow excitation of the multiple dyes without image registration shift. The microscope has two camera ports, one of which has an intensified CCD camera (Quantex Corp., Sunnyvale, CA) for sensitive high-speed video image display, which is used for finding interesting areas on a slide as well as for focusing. The other camera port has a cooled CCD camera (model 200 by Photometrics Ltd., Tucson, AZ), which is used for the actual image acquisition at high resolution and sensitivity. The cooled CCD camera is interfaced to a SUN 4/330 workstation (SUN Microsystems, Inc., Mountain View, CA) through a VME bus. The entire acquisition of multicolor images is controlled using an image processing software package SCIL-Image (Delft Centre for Image Processing, Delft, Netherlands).

### Theranosis

Methods for theranosis are also provided. The methods involve processes, such as diagnostic tests, in particular diagnostic tests employing FISH, e.g., multiplex FISH, employing an above-described cell support, used to tailor therapy for a patient. Through theranosis the suitability of a drug for treatment of a particular patient can be assessed. In addition, among others, the efficacy of treatment of the patient with the drug can be assessed. For example, if an interrogated locus or loci is/are aberrant (e.g., increase or decrease in copy number or translocation, etc.) in cells prepared from a patient specimen, the tumor is likely sensitive to an active agent, such as a small molecule, a targeted monoclonal antibody (mAb), a targeted polyclonal antibody (pAb), or other active agent, in which case the active agent can, and probably should, be administered to the patient. If a chromosomal abnormality indicative of sensitivity to an active agent is not detected, the patient may not respond or may respond unfavorably to treatment with the active agent. If a decrease in the chromosomal abnormality is detected after the patient has been treated with the active agent, the treatment of the patient with the active agent may be considered effective. If an increase in the chromosomal abnormality is detected after the patient has been treated with the active agent, the treatment of the patient with the active agent may be considered ineffective. If no change in the chromosomal abnormality is detected after the patient has been treated with the active agent, the treatment of the patient with the active agent may or may not be considered effective.

In one embodiment, a cell support, which comprises at least two discrete areas, can be used. After cells, which have been prepared in accordance with the above methods, have been applied to the discrete areas, each area is contacted with at least one detectably labeled probe and, if an area is contacted with two or more detectably labeled probes, the detectably labeled probes can be distinguished. For example, each discrete area can be separately contacted with a mixture of detectably labeled probes, wherein the mixture comprises:
--a two-color break-apart probe for ALK and a two-color break-apart probe for ROSI,
--a two-color break-apart probe for ALK, a two-color break-apart probe for ROS1, and a probe for c-MET,
--a two-color break-apart probe for ALK, a two-color break-apart probe for ROSI, and a two-color break-apart probe for RET,
--a two-color break-apart probe for RET and a two-color break-apart probe for NTRK1,
--a two-color break-apart probe for RET and a probe for KIF5B,
--a probe for MET, a probe for EGFR, and a CEP for chromosome 7,
--a probe for AURKA, a locus-specific probe for 9p21, a CEP for chromosome 3, a CEP for chromosome 6, and a CEP for chromosome 7,
--a probe for MYC, a probe for EGFR, a probe for DCC, a locus-specific probe for 13q14, and a CEP for chromosome 18,
--a probe for MYC, a probe for TERC, and a CEP for chromosome 7,
--a probe for MYC, a probe for HER2, a probe for ZNF217, and a locus-specific probe for 9p21,
--a probe for MCL1, a probe for FGFR2, a probe for MET, a probe for EGFR, and a CEP for chromosome 7,
--a probe for FGFR2, a probe for PIK3CA, a CEP for chromosome 3, and a CEP for chromosome 10,
--a probe for PTEN, a probe for ERG, and a CEP for chromosome 10,
--a probe for FGFR1, a probe for FGFR2, a two-color break-away probe for FGFR2, a CEP for chromosome 8, and a CEP for chromosome 10, or
--a probe for MET, a probe for PIK3CA, a probe for EGFR, a CEP for chromosome 7, and a CEP for chromosome 3.

All of the aforementioned embodiments of the cell support can be used for theranosis of a patient's response to an active agent that targets the tyrosine kinase pathway in accordance with the methods described herein. For example, if an interrogated locus or loci is/are aberrant (e.g., increase or decrease in copy number or translocation, etc.) in cells prepared from a patient specimen, the tumor is likely sensitive to an active agent that targets the tyrosine kinase pathway, such as a small molecule tyrosine kinase inhibitor (TKI), a targeted mAb, a targeted pAb, or other active agent, in which case the active agent can, and probably should, be administered to the patient. If a chromosomal abnormality indicative of sensitivity to an active agent is not detected, the patient may not respond or may respond unfavorably to treatment with the active agent. If a decrease in the chromosomal abnormality is detected after the patient has been treated with the active agent, the treatment of the patient with the active agent may be considered effective. If an increase in the chromosomal abnormality is detected after the patient has been treated with the active agent, the treatment of the patient with the active agent may be considered ineffective. If no change in the chromosomal abnormality is detected after the patient has been treated with the active agent, the treatment of the patient with the active agent may or may not be considered effective. Examples of active agents for which a patient's response can be assessed in accordance with the above methods include, but are not limited to, crizotinib, volitinib, gefitinib, erlotinib, sorafenib, sunitinib, dasatinib, trastuzumab, and pertuzumab.

If a patient has colorectal adenoma, a cell support, which comprises at least five discrete areas can be used. After cells, which have been prepared in accordance with the above methods have been applied to the discrete areas, each area is contacted with at least one detectably labeled probe and, if an area is contacted with two or more detectably labeled probes, the detectably labeled probes can be distinguished. For example, a first area is contacted with a locus-specific probe for EGFR, a second area is contacted with a locus-specific probe for DCC, a third area is contacted with a CEP for chromosome 18, a fourth area is contacted with a locus-specific probe for 13q14 (band 14 on the q arm of chromosome 13), and a fifth area is contacted with a locus-specific probe for MYC. If desired, a sixth discrete area can be contacted with a locus-specific probe for STAT1 or a locus-specific probe for AURKA. Alternatively, a sixth discrete area can be contacted with a locus-specific probe for STAT1, and a seventh discrete area can be contacted with a locus-specific probe for AURKA. The aforementioned cell supports can be used to prognosticate risk of recurrence of colorectal adenoma and risk of progression of colorectal adenoma to colorectal adenocarcinoma. No chromosomal abnormalities indicates a low risk of recurrence of colorectal adenoma and/or a low risk of progression of colorectal adenoma to colorectal adenocarcinoma, whereas an increase in copy number of EGFR indicates an increased risk of recurrence of colorectal adenoma and/or an increased risk of progression of colorectal adenoma to colorectal adenocarcinoma, a decrease in copy number of DCC relative to CEP 18 indicates an increased risk of recurrence of colorectal adenoma and/or an increased risk of progression of colorectal adenoma to colorectal adenocarcinoma, and an increase in copy number of EGFR in further combination with a decrease in copy number of DCC relative to CEP18, an increase in copy number of 13q14, and an increase in copy number of MYC indicates a high risk of recurrence of colorectal adenoma and/or a high risk of progression of colorectal adenoma to colorectal adenocarcinoma. An increase in copy number of STAT1 and/or an increase in copy number of AURKA indicate(s) an increased risk of recurrence of colorectal adenoma and/or an increased risk of progression of colorectal adenoma to colorectal adenocarcinoma.

Cell supports, to discrete areas on the surfaces of which have been applied cells prepared in accordance with the above methods, also can be used in the following contexts:
-- If a patient has non-small cell lung cancer (NSCLC) and the patient tests positive for ALK, crizotinib (Xalkori®) can be administered to the patient. If a patient has NSCLC and the patient tests positive for ROS1, the patient may be, or may become, resistant to treatment with crizotinib.
-- Increased expression of ASCL1 and RET may be a successful prognostic biomarker for identifying smoking-related lung cancers.
-- Over-expression of AURKA, most likely due to an increase in AURKA copy number and/or chromosome 20 aneusomy, is seen in the majority of late-stage epithelial ovarian cancers and may differentially affect taxane and non-taxane-based adjuvant therapy responses (Lassmann et al., Clin. Cancer Res. 13(14): 4083-4091 (July 15, 2007)). AURKA over-expression was associated with improved overall survival in patients receiving taxol/carboplatin therapy and worse overall survival in patient receiving non-taxane-based therapy (Lassmann et al., *supra*)*.* An increase in copy number of AURKA in urothelial cells has been suggested to be a promising biomarker for the detection of bladder cancer (Park et al., J. Nat'l Cancer Inst. 100(19): 1401-1411 (Oct. 1, 2008)).
-- If a patient has chronic myeloid leukemia (CML), the patient will likely test positive for a BCR-ABL gene fusion (>95% of cases), which results from the reciprocal translocation between chromosome 9 and chromosome 22). Imatinib mesylate (marketed as Gleevec; also known as STI-571), which is a tyrosine kinase inhibitor, is administered to the patient to inhibit the ABL protein, which is hyperactive in protein signaling. Bosutinib (marketed as Bosulif) is administered to a patient, who has CML and who cannot be treated with, or who has not gotten better with, other treatment. Dasatinib (marketed as Sprycel®) is administered to a patient, who has chronic CML that is positive for a BCR-ABL gene fusion (Philadelphia chromosome) and may or may not be resistant/tolerant to prior therapy. Dasatinib is also administered to a patient, who has acute lymphoblastic leukemia (ALL) that is positive for the Philadelphia chromosome and is resistant/tolerant to prior therapy. Nilotinib (marketed as Tasigna™) is administered to a patient, who is positive for the Philadelphia chromosome and is either newly diagnosed with chronic CML or who was previously treated for chronic CML.
-- If a patient has NSCLC and the cancer over-expresses FGFR1 of FGFR2, a novel, selective FGFR inhibitor, which is known as AZD4547 and targets FGFR1, FGFR2, and FGFR3, is administered (see Cancer Lett. 307(1): 47-52 (August 2011)).
-- If a patient has breast cancer and the cancer over-expresses the HER2/neu receptor, a monoclonal antibody drug named trastuzumab (trade names include Herclon and Herceptin), which interferes with the HER2/neu receptor, is administered. If the cancer does not over-express the HER2/neu receptor, trastuzumab is not administered. If a patient has HER2-positive, metastatic breast cancer, previously received trastuzumab and a taxane, either separately or in combination, and previously received therapy for metastasis or developed disease recurrence during or within six months of completing adjuvant therapy, ado-trastuzumab emtansine (Kadcyla; Genentech) is administered.
-- Fusions of NTRK1 to normally distant genes (e.g., MPRIP or CD74) have been found in non-smokers with lung adenocarcinomas (see Vaishnavi et al., Nature Medicine 19: 1469-1472 (2013)).
-- If a patient has cancer and overexpresses RET, crizotinib (Xalkori®) or vandetanib (ZD6474) is administered. Cabozantinib (XL 184) is currently in clinical trials (Cancer Sci. 104(11): 1396-1400 (November 2013)).
-- If a patient has cancer and overexpresses ROS, crizotinib (Xalkori®) is administered.

The presence of epithelial cells can be confirmed by contacting the cells in a discrete area with a labeled anti-CAM5.2 antibody (i.e., IHC).

Thus, among other methods of theranosing, a method of theranosing a patient's response to an active agent that targets the tyrosine kinase pathway is provided. The method comprises performing ISH on a cell support comprising at least two discrete areas to which cells, which are prepared as described herein, have been applied. Each area is contacted with at least one detectably labeled probe and, if an area is contacted with two or more detectably labeled probes, the detectably labeled probes can be distinguished. Each discrete area can be separately contacted with a mixture of detectably labeled probes, wherein the mixture comprises:
--a two-color break-apart probe for ALK and a two-color break-apart probe for ROSI,
--a two-color break-apart probe for ALK, a two-color break-apart probe for ROSI, and a probe for c-MET,
--a two-color break-apart probe for ALK, a two-color break-apart probe for ROSI, and a two-color break-apart probe for RET,
--a two-color break-apart probe for RET and a two-color break-apart probe for NTRK1,
--a two-color break-apart probe for RET and a probe for KIF5B,
--a probe for MET, a probe for EGFR, and a CEP for chromosome 7,
--a probe for AURKA, a locus-specific probe for 9p21, a CEP for chromosome 3, a CEP for chromosome 6, and a CEP for chromosome 7,
--a probe for MYC, a probe for EGFR, a probe for DCC, a locus-specific probe for 13q14, and a CEP for chromosome 18,
--a probe for MYC, a probe for TERC, and a CEP for chromosome 7,
--a probe for MYC, a probe for HER2, a probe for ZNF217, and a locus-specific probe for 9p21,
--a probe for MCL1, a probe for FGFR2, a probe for MET, a probe for EGFR, and a CEP for chromosome 7,
--a probe for FGFR2, a probe for PIK3CA, a CEP for chromosome 3, and a CEP for chromosome 10,
--a probe for PTEN, a probe for ERG, and a CEP for chromosome 10,
--a probe for FGFR1, a probe for FGFR2, a two-color break-away probe for FGFR2, a CEP for chromosome 8, and a CEP for chromosome 10, or
--a probe for MET, a probe for PIK3CA, a probe for EGFR, a CEP for chromosome 7, and a CEP for chromosome 3.

In this regard, the methods of performing ISH and IHC described herein can be used in the context of any method of theranosis. Given the advantages attendant the methods described herein, their use in the context of theranosis offers improvements over currently available methods.

The above methods can be used in the assessment of potential responsiveness to treatment with a therapeutic agent, monitoring recurrence, and assessment of the efficacy of prophylactic or therapeutic treatment. Thus, the methods can aid in treatment decisions, such as the selection of a treatment agent for therapy or prophylaxis, the selection of active surveillance or therapy over surgery, and the decision to employ adjuvant treatment(s) including the selection thereof. If desired, the methods described herein can be used in conjunction with other tests, such as routine cytology, histology, antigen assay, nomogram, methylation, mutation, and the like. The methods also can be used to confirm results previously or simultaneously obtained with other prognostic methods.

For example, if, during the course of performing an above-described method of prognosticating colorectal adenoma in a patient, it is determined that an increased copy number of EGFR is present, the decision could be made to treat the patient with an anti-EGFR agent. For example, an anti-EGFR antibody, such as panitumumab (Vectibix), which is a human IgG2 monoclonal antibody (mAb) that binds to human EGFR and reportedly has been shown to block binding of epidermal growth factor (EGF) to EGFR, thereby blocking receptor signaling, and inhibiting tumor cell activation and proliferation (see, e.g., Int'l Pat. App. Pub. No. WO 98/50433 and U.S. Pat. No. 6,235,883), or cetuximab (Erbitux), which is a chimeric mAb approved for treatment of KRAS mutation-negative, EGFR-expressing, metastatic colorectal cancer, could be administered to the patient to inhibit the progression of adenoma to adenocarcinoma and/or inhibit the recurrence of adenoma. Additionally or alternatively, the decision could be made to perform a follow-up colonoscopy on the patient in about one year.

Similarly, if, during the course of performing an above-described method of prognosticating colorectal adenoma in a patient, it is determined that a decreased copy number of DCC is present, the decision could be made to treat the patient with an alternative therapy, such as an anti-cancer therapy that does not involve an anti-EGFR agent. Examples of such alternative therapies include, but are not limited to, fluorouracil, cisplatin, doxorubicin, and cyclophosphamide. Additionally or alternatively, the decision could be made to perform a follow-up colonoscopy on the patient in about one year.

Similarly, if adenoma is discovered during the course of performing a colonoscopy on the patient and the adenomatous polyp(s) is/are removed, an above-described method could be used to monitor the patient for recurrence of adenoma. Likewise, if, during the course of performing an above-described method of prognosticating colorectal adenoma in a patient, it is determined that the patient is at low risk of recurrence of adenoma, such as when it is determined that there are no chromosomal abnormalities, the decision could be made to perform a follow-up colonoscopy on the patient in about five years.

If, during the course of performing an above-described method of prognosticating colorectal adenoma in a patient, it is determined that increased copy numbers of EGFR, MYC, and 13q14 and a decreased copy number of DCC relative to CEP18 are present, the decision could be made to treat the patient with an anti-EGFR agent, such as penitumumab and/or cetuximab, alone or in combination with an alternative or adjuvant therapy, such as fluorouracil, cisplatin, doxorubicin, and/or cyclophosphamide. Additionally, the decision could be made to perform a follow-up colonoscopy on the patient in about six months. Also additionally or alternatively, an above-described method could be used to assess efficacy of treatment by periodically performing the method over time and comparing the results over time, such as from one point in time to a subsequent or prior point in time.

If an above-described method is used to assess efficacy of treatment of the patient from whom the test sample was obtained, the method optionally further comprises modifying the therapeutic/prophylactic treatment of the patient as needed to improve efficacy. The method can be adapted for use in an automated system or a semi-automated system.

Generally speaking, when monitoring disease progression and/or treatment, the chromosomal abnormality (presence or level) may be "unchanged," "favorable" (or "favorably altered"), or "unfavorable" (or "unfavorably altered"). "Elevated" or "increased" refers to a level of chromosomal abnormality in a sample of cells (e.g., epithelial cells) obtained from a patient specimen (e.g., an adenoma) that is higher than a normal or typical level or range, or is higher than another reference level or range (e.g., earlier or baseline sample). The term "lowered" or "reduced" refers to a level of chromosomal abnormality in a sample of cells (e.g., epithelial cells) from a patient specimen (e.g., an adenoma) that is lower than a normal or typical level or range, or is lower than another reference level or range (e.g., earlier or baseline sample). The term "altered" refers to a level of chromosomal abnormality in a sample of cells (e.g., epithelial cells) from a patient specimen (e.g., an adenoma) that is altered (increased or decreased) over a normal or typical level or range, or over another reference level or range (e.g., earlier or baseline sample).

The normal or typical level or range for a given chromosomal abnormality is defined in accordance with standard practice. Because the levels of chromosomal abnormalities in some instances will be very low, a so-called altered level or alteration can be considered to have occurred when there is any net change as compared to the typical or normal level or range, or reference level or range, which cannot be explained by experimental error or sample variation. Thus, the level measured in a particular sample will be compared with the level or range of levels determined in similar samples from a so-called normal subject. In this context, a "normal subject" is an individual with no detectable disease, and a "normal" or "control" patient or population is/are one(s) that exhibit(s) no detectable disease, respectively, for example. Furthermore, given that chromosomal abnormalities are not routinely found at high levels in the majority of the human population, a "normal subject" can be considered an individual with no substantial detectable increased level of a given chromosomal abnormality, and a "normal" (sometimes termed "control") patient or population is/are one(s) that exhibit(s) no substantial detectable increased level of a given chromosomal abnormality. An "apparently normal subject" is one in which chromosomal abnormalities have not been or are being assessed. The level of a given chromosomal abnormality is said to be "elevated" when the chromosomal abnormality is normally undetectable, but is detected in a test sample, as well as when the analyte is present in the test sample at a higher than normal level.

The method can also involve the detection of other markers and the like.

Thus, when monitoring the efficacy of treatment of a condition, such as cancer (e.g., adenocarcinoma), in a patient, the method can comprise the steps of:
(a) determining chromosomal abnormalities in a sample of cells (e.g., epithelial cells) from a patient specimen (e.g., an adenocarcinoma) from a subject prior to treatment with a therapeutic agent;
(b) determining the levels of chromosomal abnormalities in a later sample of cells (e.g., epithelial cells) from a patient specimen (e.g., an adenocarcinoma) from the subject after treatment with the therapeutic agent; and
(c) comparing the levels of chromosomal abnormalities as determined in step (b) with the levels of chromosomal abnormalities as determined in step (a), wherein if the levels in step (b) are unchanged or unfavorable when compared to the levels determined in step (a), then the condition, such as cancer (e.g., adenocarcinoma), is determined to have continued, progressed or worsened in the subject. By comparison, if the levels as determined in step (b) are favorable when compared to the levels as determined in step (a), then the condition, such as cancer (e.g., adenocarcinoma), is likely to have discontinued, regressed or improved in the subject. Optionally, the method further comprises treating the subject, e.g., with one or more other therapeutic agents, radiation, and/or hormone therapy, for a period of time if the comparison shows that the levels as determined in step (b), for example, are unfavorably altered with respect to the levels determined in step (a). The period of time that the subject is treated can be determined by one skilled in the art (for example, the period of time can be from about seven (7) days to about two years, preferably from about fourteen (14) days to about one (1) year).

During the course of treatment, second and subsequent samples of adenocarcinoma cells are then obtained from the subject. The number of samples and the time in which said samples are obtained from the subject are not critical. For example, a second sample could be obtained seven (7) days after the subject is first treated, a third sample could be obtained two (2) weeks after the subject is first treated, a fourth sample could be obtained three (3) weeks after the subject is first treated, a fifth sample could be obtained four (4) weeks after the subject is first treated, etc.

After each second or subsequent sample is obtained from the subject, the levels of chromosomal abnormalities in the second or subsequent sample are determined (e.g., using the methods described herein or as known in the art). The levels as determined in each of the second and subsequent samples are then compared with the levels as determined in the first sample (e.g., the sample that was originally optionally compared to the predetermined level). If the levels as determined in step (c) are favorable when compared to the levels as determined in step (a), then the condition, such as cancer (e.g., adenocarcinoma), is likely to have discontinued, regressed or improved, and the subject should continue to be treated. However, if the levels determined in step (c) are unchanged or unfavorable when compared to the levels as determined in step (a), then the condition, such as cancer (e.g., adenocarcinoma), is determined to have continued, progressed or worsened, and the subject should be treated with a higher dosage of pharmaceutical composition, radiation, or hormone, for example, or the subject should be treated differently.

Generally, for assays in which repeat testing may be done (e.g., monitoring disease progression and/or response to treatment), a second or subsequent test sample is obtained at a period in time after the first test sample has been obtained from the subject. Specifically, a second test sample from the subject can be obtained minutes, hours, days, weeks or years after the first test sample has been obtained from the subject. For example, the second test sample can be obtained from the subject at a time period of about 1 minute, about 5 minutes, about 10 minutes, about 15 minutes, about 30 minutes, about 45 minutes, about 60 minutes, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 2 days, about 3 days, about 4 days, about 5 days, about 6 days, about 7 days, about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, about 24 weeks, about 25 weeks, about 26 weeks, about 27 weeks, about 28 weeks, about 29 weeks, about 30 weeks, about 31 weeks, about 32 weeks, about 33 weeks, about 34 weeks, about 35 weeks, about 36 weeks, about 37 weeks, about 38 weeks, about 39 weeks, about 40 weeks, about 41 weeks, about 42 weeks, about 43 weeks, about 44 weeks, about 45 weeks, about 46 weeks, about 47 weeks, about 48 weeks, about 49 weeks, about 50 weeks, about 51 weeks , about 52 weeks, about 1.5 years, about 2 years, about 2.5 years, about 3.0 years, about 3.5 years, about 4.0 years, about 4.5 years, about 5.0 years, about 5.5. years, about 6.0 years, about 6.5 years, about 7.0 years, about 7.5 years, about 8.0 years, about 8.5 years, about 9.0 years, about 9.5 years or about 10.0 years after the first test sample from the subject is obtained.

Moreover, the present disclosure also relates to methods of determining whether a subject predisposed to or suffering from a condition, such as cancer (e.g., adenocarcinoma) or adenoma, will benefit from treatment. In particular, the disclosure relates to companion diagnostic methods and products. Thus, the method of "monitoring the treatment of disease in a subject" as described herein further optimally also can encompass selecting or identifying candidates for therapy. Generally, the subject is one who has experienced some symptom of the disease or who has actually been diagnosed as having, or being at risk for, such a disease, and/or who demonstrates unfavorable levels of chromosomal abnormalities, as described herein.

The method optionally comprises an assay as described herein, where levels of chromosomal abnormalities are assessed before and following treatment of a subject. The observation of unfavorable levels of chromosomal abnormalities following treatment confirms that the subject will not benefit from receiving further or continued treatment, whereas the observation of favorable levels of chromosomal abnormalities following treatment confirms that the subject will benefit from receiving further or continued treatment. This confirmation assists with management of clinical studies, and provision of improved patient care.

### Probes

An example of a probe for ALK is Vysis LSI ALK dual color break apart rearrangement probe, which is available from Abbott Molecular, Inc., Des Plaines, IL. One part of the probe, which hybridizes 3' to the ALK gene, is ∼300 kb. The other part of the probe, which hybridizes to the ALK gene (the 3' end), is approximately ∼442 kb.

An example of a probe for CDKN2 is the Vysis LSI CDKN2A spectrum orange/CEP 9 spectrum green probe set. The CDKN2a spectrum orange probe is ∼222 kb.

A break apart probe for ERG is available from Empire Genomics (Buffalo, NY). The orange part of the probe is ∼181 kb, whereas the green part of the probe is ∼189 kb. Another split probe, which employs Texas Red and FITC, is available from Abnova (Walnut, CA).

An FGFR1 FISH probe, which is orange, is available from Empire Genomics. The probe is ∼193 kb.

An FGFR2 FISH probe, which is orange, is available from Empire Genomics. The probe is ∼174 kb.

An example of a HER2 probe is LSI HER-2, which is available from Abbott Molecular, Inc. The probe is orange and ∼190 kb.

An example of a MET probe is LSI Met SpectrumRed, which is available from Abbott Molecular, Inc. The probe is red and ∼456 kb.

MCL1 FISH probes are available in five different colors from Empire Genomics.

An NTRK1 split probe is available from Abnova. One probe is labeled with Texas Red and ∼420 kb, whereas the other probe is labeled with FITC and ∼780 kb.

An example of a PIK3CA probe is LSI PIK3CA SpectrumGreen Probe from Abbott Molecular, Inc. The probe is ∼760 kb.

An example of a PTEN probe is LSI PTEN SpectrumOrange Probe from Abbott Molecular, Inc. The probe is ∼368 kb.

A RET break apart probe is available from Abbott Molecular, Inc. One probe is red and ∼469 kb. The other probe is green and ∼545 kb.

An example of a ROS probe is LSI ROS1 (Tel) SpectrumOrange Probe from Abbott Molecular, Inc. The probe is ∼317 kb.

An example of a TERC probe is LSI TERC SpectrumGold Probe from Abbott Molecular, Inc. The probe is ∼495 kb.

An example of a ZNF217 probe is LSI ZNF217 SpectrumOrange Probe from Abbott Molecular, Inc. The probe is ∼433 kb.

An example of a probe for EGFR is Vysis LSI EGFR, which is available from Abbott Molecular, Inc., Des Plaines, IL. The EGFR gene is ∼188 kb. The probe extends beyond the gene for ∼1 kb in the 5' direction and ∼106 kb in the 3' direction. The entire probe is approximately 300 kb.

An example of a probe for DCC is Abnova DCC. Abnova DCC is manufactured by Abnova Corporation, Taiwan, and is distributed by Thermo Fisher Scientific Inc., Pittsburgh, PA, under catalog number 89-028-963.

An example of a probe for 13q14 or RBI is Vysis LSI 13 (13q14), which is available from Abbott Molecular, Inc., Des Plaines, IL. LSI 13 (13q14) consists of a set of overlapping clones that contain the RBI gene and flanking regions. The RB1 gene is 180 kb. The probe extends beyond the gene for 110-170 kb in the 5' direction and approximately 120 kb in the 3' direction. The entire probe is approximately 440 kb in size and hybridizes to 7p11 region.

An example of a probe for MYC is Vysis LSI MYC (8q24), which is available from Abbott Molecular, Inc., Des Plaines, IL. The MYC gene is 4.4 kb. The probe extends beyond the gene for ∼382 kb in the 5' direction and ∼434 kb in the 3' direction. The entire probe is approximately 821 kb and hybridizes to the 8q24 region.

A probe for STAT1 can be prepared in accordance with methods known to those of ordinary skill in the art using sequence information available in the art. See, e.g., the reference sequence of Accession No. NG_008294.1, which is available from GenBank (www.ncbi.nlm.nih.gov/genbank), and Haddad et al., Cytogenet. Cell Genet. 83(1-2): 58-59 (1998), which is cited therein. Additional sequence information is also available by way of the BAC clone RP11-629B4, which is available from GenBank as Accession No. AC067945.4.

An example of a probe for AURKA is the Vysis AURKA FISH probe, which is available from Abbott Molecular, Inc., Des Plaines, IL.

Suitable probes for use as chromosomal probes hybridize with repetitive DNA associated with the centromere of a chromosome. Centromeres of primate chromosomes contain a complex family of long-tandem repeats of DNA, which are composed of a monomer repeat length of about 171 base pairs (bp), that is referred to as α-satellite DNA. Chromosomal probes are typically about 50-1x10⁵ nucleotides in length. Longer probes typically comprise smaller fragments of about 100-500 nucleotides in length.

Chromosome enumerator probes (CEP) and locus-specific probes that target a chromosome region or sub-region can be obtained commercially or readily prepared by those in the art. Such probes can be commercially obtained from Abbott Molecular, Inc. (Des Plaines, IL), Molecular Probes, Inc. (Eugene, OR), or Cytocell (Oxfordshire, UK). Chromosomal probes can be prepared, for example, from protein nucleic acids (PNA), cloned human DNA such as plasmids, bacterial artificial chromosomes (BACs), and PI artificial chromosomes (PACs) that contain inserts of human DNA sequences. A region of interest can be obtained via PCR amplification or cloning. In another embodiment, the chromosomal probes can be oligo probes. Alternatively, chromosomal probes can be prepared synthetically in accordance with methods known in the art.

When targeting of a particular gene locus is desired, probes that hybridize along the entire length of the targeted gene can be preferred, although not required. A locus-specific probe can be designed to hybridize to an oncogene or tumor suppressor gene, the genetic aberration of which is correlated with metastasis, e.g., MYC.

The probes can be prepared by any method known in the art. Probes can be synthesized or recombinantly produced. Such probes can range in length from about 25,000 base pairs to about 800,000 base pairs.

Preferably, probes are detectably labeled, and, when two or more probes are used simultaneously or sequentially on the same sample, preferably each probe is distinctly labeled. Preferably, the probes are detectably labeled with fluorophores. Examples of preferred fluorophores include, but are not limited to, 7-amino-4-methylcoumarin-3-acetic acid (AMCA), 5-carboxy-X-rhodamine, 6-carboxy-X-rhodamine, lissamine rhodamine B, 5-carboxyfluorescein, 6-carboxyfluorescein, fluorescein-5-isothiocyanate (FITC), 7-diethylaminocoumarin-3-carboxylic acid, tetramethylrhodamine-5-isothiocyanate, tetramethylrhodamine-6-isothiocyanate, 5-carboxyltetramethylrhodamine, 6-carboxytetramethylrhodamine, 7-hydroxycoumarin-3-carboxylic acid, N-4,4-difluoro-5,7-dimethy-4-bora-3a,4a-diaza-3-indacenepropionic acid, eosin-5-isothiocyanate, erythrosine-5-isothiocyanate, SpectrumRed™ (Abbott Molecular, Inc.), SpectrumGold™ (Abbott Molecular, Inc.), SpectrumGreen™ (Abbott Molecular, Inc.), SpectrumAqua™ (Abbott Molecular, Inc.), SpectrumOrange™ (Abbott Molecular, Inc., Des Plaines, IL), TEXAS RED (Molecular Probes, Inc.), Lucifer yellow, and CASCADE blue acetylazide (Molecular Probes, Inc.). The particular label used is not critical; desirably, however, the particular label does not interfere with *in situ* hybridization of the probe and the detection of label on any other probe. The label desirably is detectable in as low copy number as possible to maximize the sensitivity of the assay and be detectable above any background signal. Also desirably, the label provides a highly localized signal, thereby providing a high degree of spatial resolution.

Attachment of fluorophores to nucleic acid probes is well-known in the art and can be accomplished by any available means. Fluorophores can be covalently attached to a particular nucleotide, for example, and the labeled nucleotide incorporated into the probe using standard techniques such as nick translation, random priming (Rigby et al., J. Mol. Biol. 113: 237 (1997)), PCR labeling, end labeling, direct labeling by chemical modification of particular residues, such as cytosine residues (U.S. Pat. No. 5,491,224), and the like. Alternatively, the fluorophore can be covalently attached to nucleotides with activated linker arms, which have been incorporated into the probe, for example, via a linker to the deoxycytidine nucleotides of the probe that have been transaminated. Methods for labeling probes are described in U.S. Pat. No. 5,491,224, and Morrison et al., Molecular Cytogenetics: Protocols and Applications, Chapter 2, "Labeling Fluorescence In Situ Hybridization Probes for Genomic Targets," pp. 21-40, Fan, Ed., Humana Press (2002), both of which are herein incorporated by reference for their descriptions of labeling probes.

One of skill in the art will recognize that other agents or dyes can be used in lieu of fluorophores as label-containing moieties. Luminescent agents include, for example, radioluminescent, chemiluminescent, bioluminescent, and phosphorescent label-containing moieties. Agents that are detectable with visible light include cyanin dyes. Alternatively, detection moieties that are visualized by indirect means can be used. For example, probes can be labeled with biotin or digoxygenin using routine methods known in the art, and then further processed for detection. Visualization of a biotin-containing probe can be achieved via subsequent binding of avidin conjugated to a detectable marker. The detectable marker may be a fluorophore, in which case visualization and discrimination of probes can be achieved as described below.

Chromosomal probes hybridized to target regions alternatively can be visualized by enzymatic reactions of label moieties with suitable substrates for the production of insoluble color products. Each probe can be discriminated from other probes within the set by choice of a distinct label moiety. A biotin-containing probe within a set can be detected via subsequent incubation with avidin conjugated to alkaline phosphatase (AP) or horseradish peroxidase (HRP) and a suitable substrate. 5-bromo-4-chloro-3-indolylphosphate and nitro blue tetrazolium (NBT) serve as substrates for alkaline phosphatase, while diaminobenzoate serves as a substrate for HRP.

### Kit

Also provided is a kit. The kit comprises (a) a cell support and/or at least one probe for ISH or IHC, and (b) instructions for carrying out an above-described method on a cell support comprising at least two discrete areas to which cells, which are prepared as described herein, have been applied, in which case each area is contacted with at least one detectably labeled probe and, if an area is contacted with two or more detectably labeled probes, the detectably labeled probes can be distinguished.

In an embodiment, the kit comprises (a) a set of probes that enables prognosis of colorectal adenoma in a patient and (b) instructions for prognosticating colorectal adenoma in a patient. Thus, the kit can comprise (a) a set of probes that enables prognosis of colorectal adenoma in a patient, wherein the set of probes comprises, or consists of, a locus-specific probe for EGFR, a locus-specific probe for DCC, chromosome enumeration probe for chromosome 18 (CEP18), a locus-specific probe for 13q14, and a locus-specific probe for MYC, and (b) instructions for prognosticating colorectal adenoma in a patient, wherein the instructions comprise determining in a sample (such as a sample of cells, e.g., epithelial cells) of a colorectal adenoma obtained from the patient the presence or absence of chromosomal abnormalities. The set of probes can further comprise one or more other locus-specific probes and/or centromeric probes (CEP), such as a locus-specific probe for STAT1 and/or a locus-specific probe for AURKA. No chromosomal abnormalities indicates a low risk of recurrence of colorectal adenoma and/or a low risk of progression of colorectal adenoma to colorectal adenocarcinoma, whereas an increase in copy number of EGFR indicates an increased risk of recurrence of colorectal adenoma and/or an increased risk of progression of colorectal adenoma to colorectal adenocarcinoma, a decrease in copy number of DCC relative to CEP18 indicates an increased risk of recurrence of colorectal adenoma and/or an increased risk of progression of colorectal adenoma to colorectal adenocarcinoma, and an increase in copy number of EGFR in further combination with a decrease in copy number of DCC relative to CEP18, an increase in copy number of 13q14, and an increase in copy number of MYC indicates a high risk of recurrence of colorectal adenoma and/or a high risk of progression of colorectal adenoma to colorectal adenocarcinoma. An increase in copy number of STAT1 and/or an increase in copy number of AURKA indicate(s) an increased risk of recurrence of colorectal adenoma and/or an increased risk of progression of colorectal adenoma to colorectal adenocarcinoma.

Such kits may further comprise blocking agents or other probes, various labels or labeling agents to facilitate detection of the probes, reagents for hybridization (e.g., buffers), a metaphase spread, and the like.

### EXAMPLES

The following examples serve to illustrate the present disclosure. The examples are not intended to limit the scope of the claimed invention in any way.

### Example 1

This example describes the isolation of epithelial cells from freshly frozen colon tissue samples and confirmation of the origin of the cells.

Frozen colon tissue samples were thawed at room temperature. The tissue was rinsed in Hank's buffer at room temperature. The tissue was then soaked in pre-warmed Hank's buffer with 20 mM DTT and 5 mM EDTA at 37 °C for five minutes. The tissue was transferred into a 10 mL Falcon tube with 2 mL of shaking solution (Hank's buffer with 20 mM DTT, 5 mM CaCl₂, 5 mM MgCl₂, and 10% DMSO at 4 °C), and the tube was vortexed for 5-10 seconds. The tissue was removed from the Falcon tube. The supernatant was either (i) strained through a cell strainer (such as a 100 µM nylon cell strainer available from Becton Dickinson) or (ii) centrifuged at 1,000 x g for 10 minutes to spin down cells, after which the supernatant was discarded and the cells were re-suspended with residual supernatant. Cell suspensions were transferred into ThinPrep vials with 20 mL PreservCyt® solution. The cells were fixed in PreservCyt® solution overnight at room temperature. The vials were stored at ambient temperature until slide preparation. If the vials needed to be stored long-term, the vials were placed at 2-8 °C.

Slides were made using a ThinPrep T-2000 processor in accordance with manufacturer's instructions. The slides were soaked in 95% ethanol for 15 minutes at room temperature and then immediately transferred into Carnoy's solution (3:1 methanol:acetic acid fixative) for 30 minutes at room temperature. Afterwards, the slides were dried in a vertical position at room temperature. If the slides needed to be stored long-term, the slides were stored at -20 °C until use.

The isolation of epithelial cells from tissue, such as fresh, fresh frozen, or formalin-fixed, paraffin-embedded (FFPE), was confirmed using an immunofluorescent anti-CAM5.2 antibody in accordance with the following procedure:
1. Rinse the slides with 1xPBS (phosphate-buffered saline).
2. Add 100 µL of 1% fresh Blocking Reagent (Invitrogen, Inc., Carlsbad, CA) prepared in PBS to the slides, and cover with Parafilm.
3. Incubate the slides in a humidified box at 37°C for 25 minutes.
4. Remove the Parafilm, and discard the reagent off the slides by tapping.
5. Apply 100 µL of CAM5.2-FITC antibody to the slides, and cover with Parafilm.
6. Incubate the slides in a humidified box at 37°C for 1 hour.
7. Wash the slides in 1xPBST (1xPBS with 0.05% Tween-20) at 37°C for 5 minutes; repeat twice.
8. Apply DAPI and cover with coverslips.
9. View slides under regular fluorescent microscope with appropriate filters.
Microscopic evaluation revealed that ≥ 95% of the isolated cells was of epithelial origin. The epithelial cells were distributed evenly within the target and presented primarily as individual cells with good cellular and nuclear morphology.

### Example 2

This example describes the fluorescence *in situ* hybridization (FISH) procedure used on the slides from Example 1.

Slides were placed in 2xSSC at 73 °C for two minutes, after which slides were incubated with pepsin (0.5 mg/mL) at 37 °C for 10 minutes. Subsequently, slides were placed in 1xPBS at room temperature for five minutes, 1% NBF at room temperature for five minutes, and 1xPBS at room temperature for five minutes. Slides were dehydrated by placement in 70% ethanol, 85% ethanol, and 100% ethanol for one minute each and then air dried. Probe (10 µl) and then a coverslip were applied to the slide. The coverslip was sealed with rubber cement. Slides were co-denatured in ThermoBrite for two minutes at 72 °C and 12-18 hours (overnight) at 37 °C. The next day slides were treated with 0.4xSSC/0.3% NP-40 for two minutes at 73 °C and 2xSSC/0.1% NP-40 for one minute at room temperature. Slides were then air dried, mounted with DAPI I anti-fade solution (Abbott Molecular, Inc., Des Plaines, IL) and covered with a coverslip.

Following hybridization, slides were evaluated using fluorescence microscopy with SpectrumGold™, SpectrumOrange™, SpectrumRed™, SpectrumGreen™, SpectrumAqua™, and DAPI filters. A cell was considered to be chromosomally abnormal if it possessed the following:
i.) gene copy number gain represented by three or more signals per cell,
ii.) and/or gene copy number loss represented by one or zero signal,
iii.) and/or the relative loss represented by the ratio of LSI probe to CEP being less than 1.0.
Fifty cells were enumerated in a designated target area on each slide, and the copy number of each probe was recorded.

Thirteen genomic regions associated with colorectal cancer (CRC) were evaluated on six CRC specimens and their respective normal adjacent tissues (NAT) to select FISH probes that had the highest frequency of chromosomal aberrations. Based on this assessment, five candidate probes (EGFR 7p12, MYC 8q24, 13q14, DCC 18q21, and CEP18) were selected for additional testing on a set of 27 frozen tissue specimens diagnosed with colorectal adenoma and 3 normal adjacent tissues. Of the 27 tested cases, 11 patients were diagnosed with concurrent colon cancer, whereas 16 patients with adenoma did not have concurrent colon cancer. For each specimen, FISH patterns were recorded for fifty cells/nuclei by one observer, and the number of chromosomally abnormal cells was evaluated. A cutoff point of at least 10 FISH abnormal cells was used to classify FISH positivity. A cell was considered as abnormal when it had copy number gains for EGFR, MYC and 13q14 probes and copy number loss for DCC relative to CEP 18. The results showed that there were four distinct FISH patterns identified in colorectal adenoma cases: all four probes having no aberrations (disomic); only DCC gene loss; only EGFR gene gain; and all four probes abnormal, i.e., MYC, 13q14, and EGFR amplification and DCC deletion (see Table 1).

**Table 1**

| | FISH negative | FISH positive | | |
|---|---|---|---|---|
| Total Adenoma (27) | all FISH probes normal | DCC loss only | EGFR gain only | All FISH probes abnormal |
| Concurrent Cancer (11) | 9.1% (1/11) | 36.4% (4/11) | 18.2% (2/11) | 36.4% (4/11) |
| No Concurrent Cancer (16) | 62.5% (10/16) | 0.0% (0/16) | 18.8% (3/16) | 18.8% (3/16) |

In this limited set of samples, 91% of adenoma specimens obtained from patients with concurrent colorectal cancer were found to be FISH-positive, whereas only 38% of specimens were FISH-positive when obtained from patients who did not have concurrent colorectal cancer presented.

The observations suggest that different genetic profiles in adenoma specimens may have clinical value for personalized medicine. Patients with no FISH abnormalities (FISH pattern 1) may have the lowest risk of polyp recurrence and/or progression and have the best prognosis and, therefore, do not require treatment. For such patients, a follow-up colonoscopy, such as five years later, could be recommended. In contrast, patients with colonic adenomatous polyps abnormal for all probes (FISH pattern 4) could be at the highest risk of polyp recurrence and/or progression and, therefore, require treatment (e.g., anti-EGFR agent). For such patients, a follow-up colonoscopy, such as six months later, could be recommended. EGFR amplification (FISH pattern 2) may have an increased risk of polyp recurrence and/or progression and, therefore, could benefit from targeted drug therapy (e.g., anti-EGFR agent). For such patients, a follow-up colonoscopy, such as one year later, could be recommended. A unique group of patients with the sole aberration of the DCC gene loss (FISH pattern 3) may have an increased risk of polyp recurrence and/or progression and, therefore, could benefit from another therapy and may have poor prognosis. For such patients, a follow-up colonoscopy, such as one year later, could be recommended.

All patents, patent application publications, journal articles, textbooks, and other publications mentioned in the specification are indicative of the level of skill of those in the art to which the disclosure pertains. All such publications are incorporated herein by reference to the same extent as if each individual publication were specifically and individually indicated to be incorporated by reference.

The invention illustratively described herein may be suitably practiced in the absence of any element(s) or limitation(s), which is/are not specifically disclosed herein. Thus, for example, each instance herein of any of the terms "comprising," "consisting essentially of," and "consisting of" may be replaced with either of the other two terms. Likewise, the singular forms "a," "an," and "the" include plural references unless the context clearly dictates otherwise. Thus, for example, references to "the method" includes one or more methods and/or steps of the type, which are described herein and/or which will become apparent to those ordinarily skilled in the art upon reading the disclosure.

The terms and expressions, which have been employed, are used as terms of description and not of limitation. In this regard, where certain terms are set forth under "Terms" and are otherwise defined, described, explained, or discussed elsewhere in the "Detailed Description," all such definitions, descriptions, explanations, and discussions are intended to be attributed to such terms. There also is no intention in the use of such terms and expressions of excluding any equivalents of the features shown and described or portions thereof. Furthermore, while subheadings, e.g., "Terms," are used in the "Detailed Description," such use is solely for ease of reference and is not intended to limit any disclosure made in one section to that section only; rather, any disclosure made under one subheading is intended to constitute a disclosure under each and every other subheading.

It is recognized that various modifications are possible within the scope of the claimed invention. Thus, it should be understood that, although the present invention has been specifically disclosed in the context of preferred embodiments and optional features, those skilled in the art may resort to modifications and variations of the concepts disclosed herein. Such modifications and variations are considered to be within the scope of the invention as claimed herein.

The present invention is further defined by the following items:
1. A method of preparing cells from a patient specimen comprising a tissue or a clump of cells, which method comprises:
   (i) soaking the patient specimen in a pre-warmed, balanced salt solution comprising a reducing agent and a chelating agent;
   (ii) transferring the patient specimen to a fresh, balanced salt solution comprising a reducing agent, a source of calcium ions, and a polar aprotic solvent and mixing;
   (iii) removing the patient specimen from the fresh, balanced salt solution and either (a) centrifuging the solution to pellet disaggregated cells or (b) straining the solution to collect disaggregated cells; and
   (iv) after (iii)(a), discarding the supernatant, re-suspending the pelleted cells in residual supernatant, and placing the re-suspended cells in a fixative or, after (iii)(b), placing the strained cells in a fixative;
   whereupon cells from a patient specimen comprising a tissue or a clump of cells are prepared.
2. The method of item 1, wherein the patient specimen is (a) a fresh specimen, (b) a thawed, freshly frozen specimen, or (c) a partially fixed specimen.
3. The method of item 1 or 2, which further comprises rinsing the patient specimen in a balanced salt solution prior to (i).
4. The method of item 3, which comprises:
   --rinsing the patient specimen in Hank's buffer at room temperature,
   --soaking the patient specimen in pre-warmed Hank's buffer containing 20 mM DTT (dithiothrcitol) and 5 mM EDTA (ethylenediaminetetraacetic acid) at 37 °C for around five minutes,
   --transferring the rinsed and soaked patient specimen to fresh Hank's buffer containing 20 mM DTT, 5 mM CaCl₂, and 10% DMSO (dimethylsulfoxide), and mixing for about 5 seconds to about 10 seconds,
   --removing the patient specimen from the fresh Hank's buffer and either (a) centrifuging the solution at about 1000 x g for about 10 minutes to pellet disaggregated cells or (b) straining the solution to collect disaggregated cells, and
   --either, after centrifuging, discarding the supernatant, re-suspending the pelleted cells in residual supernatant, and placing the re-suspended cells in a solution or, after straining, placing the strained cells in a solution, wherein the solution comprises from about 30 wt.% to about 60 wt.% methanol, from about 40 wt.% to about 70 wt.% water, a buffer, and a preservative and wherein the solution is kept at room temperature overnight.
5. The method of item 1, 2, 3 or 4, which further comprises: (v) applying the cells to a cell support.
6. The method of item 5, wherein the cell support is a cytology slide, and wherein the method further comprises:
   (vi) soaking the slide in a water-alcohol solution,
   (vii) soaking the slide in a fixative, and
   (viii) drying the slide.
7. The method of item 6, wherein the water-alcohol solution is 95% ethanol and the slide is soaked in the ethanol for about 15 minutes at room temperature, wherein the fixative is Carnoy's solution and the slide is soaked in the Carnoy's solution for about 30 minutes at room temperature, and wherein the slide is dried at room temperature.
8. A method of performing *in situ* hybridization (ISH) or immunohistochemistry (IHC), which method comprises contacting cells, which have been prepared in accordance with the method of item 1, 2, 3 or 4, with a detectably labeled probe under hybridizing conditions and visualizing the detectable label in less than about 14 hours of removal of the specimen from the patient.
9. The method of item 8, wherein the detectably labeled probe is a fluorescently labeled nucleic acid probe or a fluorescently labeled antibody (or antigenically reactive fragment thereof) probe.
10. The method of item 8, wherein the cells have been applied to a cell support.
11. The method of item 10, wherein the cell support comprises at least two discrete areas and the cells have been applied to the discrete areas, in which case each area is contacted with at least one detectably labeled probe and, if an area is contacted with two or more detectably labeled probes, the detectably labeled probes can be distinguished.
12. The method of item 11, wherein each discrete area is separately contacted with a mixture of detectably labeled probes, wherein the mixture comprises:
   --a two-color break-apart probe for ALK and a two-color break-apart probe for ROSI,
   --a two-color break-apart probe for ALK, a two-color break-apart probe for ROS1, and a probe for c-MET,
   --a two-color break-apart probe for ALK, a two-color break-apart probe for ROS1, and a two-color break-apart probe for RET,
   --a two-color break-apart probe for RET and a two-color break-apart probe for NTRK1,
   --a two-color break-apart probe for RET and a probe for KIF5B,
   --a probe for MET, a probe for EGFR, and a CEP for chromosome 7,
   --a probe for AURKA, a locus-specific probe for 9p21, a CEP for chromosome 3, a CEP for chromosome 6, and a CEP for chromosome 7,
   --a probe for MYC, a probe for EGFR, a probe for DCC, a locus-specific probe for 13q14, and a CEP for chromosome 18,
   --a probe for MYC, a probe for TERC, and a CEP for chromosome 7,
   --a probe for MYC, a probe for HER2, a probe for ZNF217, and a locus-specific probe for 9p21,
   --a probe for MCL1, a probe for FGFR2, a probe for MET, a probe for EGFR, and a CEP for chromosome 7,
   --a probe for FGFR2, a probe for PIK3CA, a CEP for chromosome 3, and a CEP for chromosome 10,
   --a probe for PTEN, a probe for ERG, and a CEP for chromosome 10,
   --a probe for FGFR1, a probe for FGFR2, a two-color break-away probe for FGFR2, a CEP for chromosome 8, and a CEP for chromosome 10, or
   --a probe for MET, a probe for PIK3CA, a probe for EGFR, a CEP for chromosome 7, and a CEP for chromosome 3.
13. A method of performing *in situ* hybridization (ISH) or immunohistochemistry (IHC), which method comprises contacting cells, which have been disaggregated from a specimen selected from the group consisting of a fine needle aspiration, a core needle biopsy, an excisional biopsy, and a resection, with a detectably labeled probe under hybridizing conditions and visualizing the detectable label in less than about 14 hours of removal of the specimen from the patient.
14. The method of item 13, wherein the detectably labeled probe is a fluorescently labeled nucleic acid probe or a fluorescently labeled antibody (or antigenically reactive fragment thereof) probe.
15. The method of item 13, wherein the cells have been applied to a cell support.
16. The method of item 15, wherein the cell support comprises at least two discrete areas and the cells have been applied to the discrete areas, in which case each area is contacted with at least one detectably labeled probe and, if an area is contacted with two or more detectably labeled probes, the detectably labeled probes can be distinguished.
17. The method of item 16, wherein each discrete area is separately contacted with a mixture of detectably labeled probes, wherein the mixture comprises:
   --a two-color break-apart probe for ALK and a two-color break-apart probe for ROSI,
   --a two-color break-apart probe for ALK, a two-color break-apart probe for ROS1, and a probe for c-MET,
   --a two-color break-apart probe for ALK, a two-color break-apart probe for ROS1, and a two-color break-apart probe for RET,
   --a two-color break-apart probe for RET and a two-color break-apart probe for NTRK1,
   --a two-color break-apart probe for RET and a probe for KIF5B,
   --a probe for MET, a probe for EGFR, and a CEP for chromosome 7,
   --a probe for AURKA, a locus-specific probe for 9p21, a CEP for chromosome 3, a CEP for chromosome 6, and a CEP for chromosome 7,
   --a probe for MYC, a probe for EGFR, a probe for DCC, a locus-specific probe for 13q14, and a CEP for chromosome 18,
   --a probe for MYC, a probe for TERC, and a CEP for chromosome 7,
   --a probe for MYC, a probe for HER2, a probe for ZNF217, and a locus-specific probe for 9p21,
   --a probe for MCL1, a probe for FGFR2, a probe for MET, a probe for EGFR, and a CEP for chromosome 7,
   --a probe for FGFR2, a probe for PIK3CA, a CEP for chromosome 3, and a CEP for chromosome 10,
   --a probe for PTEN, a probe for ERG, and a CEP for chromosome 10,
   --a probe for FGFR1, a probe for FGFR2, a two-color break-away probe for FGFR2, a CEP for chromosome 8, and a CEP for chromosome 10, or
   --a probe for MET, a probe for PIK3CA, a probe for EGFR, a CEP for chromosome 7, and a CEP for chromosome 3.
18. A method of theranosing a patient's response to treatment with an active agent that targets the tyrosine kinase pathway, which method comprises performing ISH or IHC according to the method of item 12.
19. The method of item 18, wherein the active agent that target the tyrosine kinase pathway is crizotinib, volitinib, gefitinib, erlotinib, sorafenib, sunitinib, dasatinib, trastuzumab, or pertuzumab.
20. A method of theranosing a patient's response to treatment with an active agent that targets the tyrosine kinase pathway, which method comprises performing ISH according to the method of item 17.
21. The method of item 20, wherein the active agent that target the tyrosine kinase pathway is crizotinib, volitinib, gefitinib, erlotinib, sorafenib, sunitinib, dasatinib, trastuzumab, or pertuzumab.
22. In a method of theranosis, the improvement comprising the use of the method of item 10, 11, or 12.
23. In a method of theranosis, the improvement comprising the use of the method of item 15, 16 or 17.
24. A cell support on the surface of which are cells, which have been contacted with detectably labeled probes in accordance with the method of item 11 or 12.
25. A cell support on the surface of which are cells, which have been contacted with detectably labeled probes in accordance with the method of item 16 or 17.
26. A kit comprising:
   (a) a cell support and/or at least one probe for ISH or IHC, and
   (b) instructions for carrying out the method of item 11 or 12.
27. A kit comprising:
   (a) a cell support and/or at least one probe for ISH or IHC, and
   (b) instructions for carrying out the method of item 16 or 17.
28. A kit comprising:
   (a) a cell support and/or at least one probe for ISH or IHC, and
   (b) instructions for carrying out the method of item 18 or 19.
29. A kit comprising:
   (a) a cell support and/or at least one probe for ISH or IHC, and
   (b) instructions for carrying out the method of item 20 or 21.

## Claims

1. A method of simultaneously determining the copy number of at least two chromosomal regions in a cell suspension, which method comprises:
(a) disaggregating cells from a patient specimen to produce a cell suspension;
(b) applying the cell suspension to two or more discrete areas of a cell support;
(b) contacting each of the two or more discrete areas of the cell support containing the cell suspension applied thereto with a mixture of detectably labeled probes under hybridizing conditions, wherein each mixture comprises a different set of detectably labeled probes,
(c) performing *in situ* hybridization (ISH) following step (b) on the cell suspension applied to the two or more discrete areas of the cell support and visualizing the signal from each of the detectably labeled probes from each mixture in less than 14 hours from obtaining the patient specimen; and
(d) counting the copy number of the at least two chromosomal regions in the cell suspension, resulting in the simultaneous determination of the copy number of at least two chromosomal regions in a cell suspension.

2. The method of claim 1, wherein disaggregating cells from a patient specimen to produce a cell suspension comprises:
(i) soaking the patient specimen in a pre-warmed, balanced salt solution comprising a reducing agent and a chelating agent;
(ii) transferring the patient specimen to a fresh, balanced salt solution comprising a reducing agent, a source of calcium ions, and a polar aprotic solvent and mixing;
(iii) removing the remaining patient specimen from the balanced salt solution, and either (a) straining the solution to collect disaggregated cells and centrifuging the solution to pellet disaggregated cells or (b) only centrifuging the solution to pellet disaggregated cells;
(iv) discarding the supernatant, re-suspending the pelleted cells in residual supernatant, and placing the resuspended cells in a fixative.

3. The method of claim 2, which further comprises rinsing the patient specimen in a balanced salt solution prior to soaking the patient specimen in a pre-warmed, balanced salt solution.

4. The method of claim 3, wherein the balanced salt solution is Hank's buffer.

5. The method of claim 4, which comprises:
(i) rinsing the patient specimen in Hank's buffer at room temperature and soaking the patient specimen in pre-warmed Hank's buffer containing 20 mM DTT (dithiothreitol) and 5 mM EDTA (ethylenediaminetetraacetic acid) at 37° C for five minutes,
(ii) transferring the rinsed and soaked patient specimen to fresh Hank's buffer containing 20 mM DTT, 5 mM CaCl2, and 10% DMSO (dimethylsulfoxide) at 4° C, and mixing for about 5 seconds to about 10 seconds,
(iii) removing the patient specimen from the Hank's buffer and either (a) straining the solution to collect disaggregated cells and centrifuging the solution at about 1000×g for about 10 minutes to pellet disaggregated cells or (b) only centrifuging the solution at about 1000×g for about 10 minutes to pellet disaggregated cells, and
(iv) either, after centrifuging, discarding the supernatant, re-suspending the pelleted cells in residual supernatant, and placing the re-suspended cells in a solution wherein the solution comprises from about 30 wt. % to about 60 wt. % methanol, from about 40 wt. % to about 70 wt. % water, a buffer, and wherein the solution is kept at room temperature overnight.

6. The method of claim 5, which further comprises soaking the cell support in a water-alcohol solution, soaking the cell support in a fixative, and/or drying the cell support.

7. The method of any one of claims 1-6, wherein the patient specimen is (a) a fresh specimen, (b) a thawed, freshly frozen specimen, or (c) a partially fixed specimen.

8. The method of any one of claims 1-7, wherein the patient specimen is an adenoma or an adenocarcinoma.

9. The method of any one of claims 1-8, wherein the cell support is a cytology slide, a cyto-spin, or a microfluidic chamber.

10. The method of any one of claims 1-9, wherein the two or more discrete areas of the cell support comprise depressions, wells, or chambers.

11. The method of claim 10, wherein each mixture of two or more detectably labeled probes comprises:
(a) a two-color break-apart probe for ALK and a two-color break-apart probe for ROS1,
(b) a two-color break-apart probe for ALK, a two-color break-apart probe for ROS1, and a probe for c-MET,
(c) a two-color break-apart probe for ALK, a two-color break-apart probe for ROS1, and a two-color break-apart probe for RET,
(d) a two-color break-apart probe for RET and a two-color break-apart probe for NTRK1,
(e) a two-color break-apart probe for RET and a probe for KIF5B,
(f) a probe for MET, a probe for EGFR, and a CEP for chromosome 7,
(g) a probe for AURKA, a locus-specific probe for 9p21, a CEP for chromosome 3, a CEP for chromosome 6, and a CEP for chromosome 7,
(h) a probe for MYC, a probe for EGFR, a probe for DCC, a locus-specific probe for 13q14, and a CEP for chromosome 18,
(i) a probe for MYC, a probe for TERC, and a CEP for chromosome 7,
(j) a probe for MYC, a probe for HER2, a probe for ZNF217, and a locus-specific probe for 9p21,
(k) a probe for MCL1, a probe for FGFR2, a probe for MET, a probe for EGFR, and a CEP for chromosome 7,
(l) a probe for FGFR2, a probe for PIK3CA, a CEP for chromosome 3, and a CEP for chromosome 10,
(m) a probe for PTEN, a probe for ERG, and a CEP for chromosome 10,
(n) a probe for FGFR1, a probe for FGFR2, a two-color break-away probe for FGFR2, a CEP for chromosome 8, and a CEP for chromosome 10, or
(o) a probe for MET, a probe for PIK3CA, a probe for EGFR, a CEP for chromosome 7, and a CEP for chromosome 3.
